# EUROPEAN PATENT APPLICATION

(11) **EP 4 036 117 A1**
(43) Date of publication of application: **03.08.2022**
(21) Application number: 21153835.0
(22) Date of filing: 27.01.2021
(51) Int. Cl.: C07K 16/28, C07K 14/00, A61P 35/00, A61P 37/00

(54) **MCL-1 BINDING ALPHABODIES AND USES THEREOF**

(71) Applicant: Complix NV, 3590 Diepenbeek (BE)
(72) Inventor: LASTERS, Ignace, B-2018 Antwerpen (BE); DESMET, Johan, B-8500 Kortrijk (BE); LOVERIX, Stefan, B-1740 Ternat (BE); VANDENBROUCKE, Karen, B-8552 Moen (BE); ALARD, Philippe, B-9820 Merelbeke (BE); DEROO, Sabrina, F-57330 Roussy-le-Village (FR)
(74) Representative: De Clercq & Partners

(57) **Abstract**

Present invention provides polypeptides capable of specifically binding myeloid cell leukemia 1 (MCL-1) comprising at least one Alphabody structure characterized in that HRS2 comprises a MCL-1 binding region comprising a sequence LRXVGDXV (SEQ ID NO: 1), wherein X can be any amino acid; the polypeptide comprises an albumin binding region conjugated to the C-terminus of HRS3, wherein said albumin binding region comprises a sequence SDFYFXXINKA (SEQ ID NO: 2) and a sequence TXEXVXALKXXILXAH (SEQ ID NO: 3), wherein X can be any amino acid; HRS1, HRS2 and HRS3 form a triple-stranded, anti-parallel, alpha-helical coiled coil; and HRS1, HRS2, HRS3 and the albumin binding region together form a five-stranded alpha-helical bundle. Also provided herein is the use of these polypeptides for use in treating a neoplastic disease.

SEQUENCE LISTING

<110>  Complix NV

<120>  MCL-1 BINDING ALPHABODIES AND USES THEREOF

<130>  COMP-023-EP-PRIO1

<160>  43

<170>  PatentIn version 3.5

<210>  1
<211>  8
<212>  PRT
<213>  Artificial Sequence

<220>
<223>  MCL-1 binding region


<220>
<221>  SITE
<222>  (3)..(3)
<223>  can be any amino acid

<220>
<221>  SITE
<222>  (7)..(7)
<223>  can be any amino acid

<400>  1

Leu Arg Xaa Val Gly Asp Xaa Val
1               5


<210>  2
<211>  11
<212>  PRT
<213>  Artificial Sequence

<220>
<223>  fragment of albumin binding region


<220>
<221>  SITE
<222>  (6)..(7)
<223>  can be any amino acid

<400>  2

Ser Asp Phe Tyr Phe Xaa Xaa Ile Asn Lys Ala
1               5                   10


<210>  3
<211>  16
<212>  PRT
<213>  Artificial Sequence

<220>

<223>  fragment of albumin binding region

<220>
<221>  SITE
<222>  (2)..(2)
<223>  can be any amino acid

<220>
<221>  SITE
<222>  (4)..(4)
<223>  can be any amino acid

<220>
<221>  SITE
<222>  (6)..(6)
<223>  can be any amino acid

<220>
<221>  SITE
<222>  (10)..(11)
<223>  can be any amino acid

<220>
<221>  SITE
<222>  (14)..(14)
<223>  can be any amino acid

<400>  3

Thr Xaa Glu Xaa Val Xaa Ala Leu Lys Xaa Xaa Ile Leu Xaa Ala His
1               5                   10                  15

<210>  4
<211>  28
<212>  PRT
<213>  Artificial Sequence

<220>
<223>  albumin binding region

<220>
<221>  SITE
<222>  (6)..(7)
<223>  can be any amino acid

<220>
<221>  SITE
<222>  (14)..(14)
<223>  can be any amino acid

<220>
<221>  SITE
<222>  (16)..(16)
<223>  can be any amino acid

<220>
<221>  SITE
<222>  (18)..(18)
<223>  can be any amino acid

<220>
<221>  SITE
<222>  (22)..(23)
<223>  can be any amino acid

<220>
<221>  SITE
<222>  (26)..(26)
<223>  can be any amino acid

<400>  4

Ser Asp Phe Tyr Phe Xaa Xaa Ile Asn Lys Ala Lys Thr Xaa Glu Xaa
1               5                   10                  15

Val Xaa Ala Leu Lys Xaa Xaa Ile Leu Xaa Ala His
            20                  25

<210>  5
<211>  28
<212>  PRT
<213>  Artificial Sequence

<220>
<223>  albumin binding region

<220>
<221>  SITE
<222>  (6)..(7)
<223>  can be any amino acid

<220>
<221>  SITE
<222>  (18)..(18)
<223>  can be any amino acid

<220>
<221>  SITE
<222>  (22)..(23)
<223>  can be any amino acid

<220>
<221>  SITE
<222>  (26)..(26)
<223>  can be any amino acid

<400>  5

Ser Asp Phe Tyr Phe Xaa Xaa Ile Asn Lys Ala Lys Thr Cys Glu Ala
1               5                   10                  15

Val Xaa Ala Leu Lys Xaa Xaa Ile Leu Xaa Ala His
            20                  25

<210>  6
<211>  165
<212>  PRT
<213>  Homo sapiens

<400>  6

Thr Pro Pro Pro Ala Glu Glu Glu Glu Asp Glu Leu Tyr Arg Gln Ser
1               5                   10                  15

Leu Glu Ile Ile Ser Arg Tyr Leu Arg Glu Gln Ala Thr Gly Ala Lys
            20                  25                  30

Asp Thr Lys Pro Met Gly Arg Ser Gly Ala Thr Ser Arg Lys Ala Leu
        35                  40                  45

Glu Thr Leu Arg Arg Val Gly Asp Gly Val Gln Arg Asn His Glu Thr
    50                  55                  60

Ala Phe Gln Gly Met Leu Arg Lys Leu Asp Ile Lys Asn Glu Asp Asp
65                  70                  75                  80

Val Lys Ser Leu Ser Arg Val Met Ile His Val Phe Ser Asp Gly Val
                85                  90                  95

Thr Asn Trp Gly Arg Ile Val Thr Leu Ile Ser Phe Gly Ala Phe Val
            100                 105                 110

Ala Lys His Leu Lys Thr Ile Asn Gln Glu Ser Cys Ile Glu Pro Leu
        115                 120                 125

Ala Glu Ser Ile Thr Asp Val Leu Val Arg Thr Lys Arg Asp Trp Leu
    130                 135                 140

Val Lys Gln Arg Gly Trp Asp Gly Phe Val Glu Phe Phe His Val Glu
145                 150                 155                 160

Asp Leu Glu Gly Gly
                165

<210>  7
<211>  8
<212>  PRT
<213>  Artificial Sequence

<220>
<223>  MCL-1 binding region

<400>  7

Leu Arg Ile Val Gly Asp Gln Val
1               5

<210>  8
<211>  26

<212>  PRT
<213>  Artificial Sequence

<220>
<223>  HRS2

<400>  8

Ile Glu Glu Ile Thr Lys Gln Ile Ala Ala Ile Gln Leu Arg Ile Val
1               5                   10                  15

Gly Asp Gln Val Gln Ile Ala Tyr Gln Thr
            20                  25

<210>  9
<211>  53
<212>  PRT
<213>  Finegoldia magna

<400>  9

Thr Ile Asp Gln Trp Leu Leu Lys Asn Ala Lys Glu Asp Ala Ile Ala
1               5                   10                  15

Glu Leu Lys Lys Ala Gly Ile Thr Ser Asp Phe Tyr Phe Asn Ala Ile
            20                  25                  30

Asn Lys Ala Lys Thr Val Glu Glu Val Asn Ala Leu Lys Asn Glu Ile
        35                  40                  45

Leu Lys Ala His Ala
    50

<210>  10
<211>  10
<212>  PRT
<213>  Finegoldia magna

<400>  10

Ala Lys Glu Asp Ala Ile Ala Glu Leu Lys
1               5                   10

<210>  11
<211>  8
<212>  PRT
<213>  Finegoldia magna

<400>  11

Asp Phe Tyr Phe Asn Ala Ile Asn
1               5

<210>  12
<211>  13

<212>  PRT
<213>  Finegoldia magna

<400>  12

Glu Glu Val Asn Ala Leu Lys Asn Glu Ile Leu Lys Ala
1               5                   10

<210>  13
<211>  16
<212>  PRT
<213>  Artificial Sequence

<220>
<223>  fragment of albumin binding region

<220>
<221>  SITE
<222>  (2)..(2)
<223>  can be any amino acid

<220>
<221>  SITE
<222>  (6)..(6)
<223>  can be any amino acid

<220>
<221>  SITE
<222>  (10)..(11)
<223>  can be any amino acid

<220>
<221>  SITE
<222>  (14)..(14)
<223>  can be any amino acid

<400>  13

Thr Xaa Glu Ala Val Xaa Ala Leu Lys Xaa Xaa Ile Leu Xaa Ala His
1               5                   10                  15

<210>  14
<211>  16
<212>  PRT
<213>  Artificial Sequence

<220>
<223>  fragment of albumin binding region

<220>
<221>  SITE
<222>  (6)..(6)
<223>  can be any amino acid

<220>
<221>  SITE
<222>  (10)..(11)
<223>  can be any amino acid

<220>
<221>  SITE
<222>  (14)..(14)
<223>  can be any amino acid

<400>  14

Thr Cys Glu Ala Val Xaa Ala Leu Lys Xaa Xaa Ile Leu Xaa Ala His
1               5                   10                  15


<210>  15
<211>  28
<212>  PRT
<213>  Artificial Sequence

<220>
<223>  albumin binding region


<220>
<221>  SITE
<222>  (6)..(7)
<223>  can be any amino acid

<220>
<221>  SITE
<222>  (14)..(14)
<223>  can be any amino acid

<220>
<221>  SITE
<222>  (18)..(18)
<223>  can be any amino acid

<220>
<221>  SITE
<222>  (22)..(23)
<223>  can be any amino acid

<220>
<221>  SITE
<222>  (26)..(26)
<223>  can be any amino acid

<400>  15

Ser Asp Phe Tyr Phe Xaa Xaa Ile Asn Lys Ala Lys Thr Xaa Glu Ala
1               5                   10                  15


Val Xaa Ala Leu Lys Xaa Xaa Ile Leu Xaa Ala His
            20                  25


<210>  16
<211>  28
<212>  PRT
<213>  Artificial Sequence

<220>
<223>  albumin binding region

<220>
<221>  SITE
<222>  (6)..(7)
<223>  can be any amino acid

<220>
<221>  SITE
<222>  (16)..(16)
<223>  can be any amino acid

<220>
<221>  SITE
<222>  (18)..(18)
<223>  can be any amino acid

<220>
<221>  SITE
<222>  (22)..(23)
<223>  can be any amino acid

<220>
<221>  SITE
<222>  (26)..(26)
<223>  can be any amino acid

<400>  16

Ser Asp Phe Tyr Phe Xaa Xaa Ile Asn Lys Ala Lys Thr Cys Glu Xaa
1               5                   10                  15

Val Xaa Ala Leu Lys Xaa Xaa Ile Leu Xaa Ala His
            20                  25

<210>  17
<211>  28
<212>  PRT
<213>  Artificial Sequence

<220>
<223>  albumin binding region

<400>  17

Ser Asp Phe Tyr Phe Glu Leu Ile Asn Lys Ala Lys Thr Cys Glu Ala
1               5                   10                  15

Val Glu Ala Leu Lys Glu His Ile Leu Ala Ala His
            20                  25

<210>  18
<211>  8
<212>  PRT
<213>  Artificial Sequence

<220>
<223>  linker region

<220>
<221>  SITE
<222>  (4)..(4)
<223>  can be any amino acid

<400>  18

Glu Leu Lys Xaa Ala Gly Ile Thr
1               5


<210>  19
<211>  8
<212>  PRT
<213>  Artificial Sequence

<220>
<223>  linker region

<400>  19

Glu Leu Lys Glu Ala Gly Ile Thr
1               5


<210>  20
<211>  7
<212>  PRT
<213>  Artificial Sequence

<220>
<223>  heptad repeat unit

<400>  20

Ile Ala Ala Ile Gln Lys Gln
1               5


<210>  21
<211>  22
<212>  PRT
<213>  Artificial Sequence

<220>
<223>  HRS3

<400>  21

Thr Glu Glu Ile Gln Lys Gln Ile Ala Ala Ile Glu Thr Gln Ile Cys
1               5                   10                  15


Lys Ile Glu Ala Ala Ile
            20


<210>  22
<211>  8
<212>  PRT
<213>  Artificial Sequence

<220>

<223>  linker region

<400>  22

Gly Gly Ser Gly Gly Ser Gly Gly
1               5


<210>  23
<211>  8
<212>  PRT
<213>  Artificial Sequence

<220>
<223>  linker region

<400>  23

Gly Ser Gly Gly Gly Gly Ser Gly
1               5


<210>  24
<211>  11
<212>  PRT
<213>  Artificial Sequence

<220>
<223>  linker region

<400>  24

Thr Gly Gly Ser Gly Gly Ser Gly Gly Met Ser
1               5                   10


<210>  25
<211>  19
<212>  PRT
<213>  Artificial Sequence

<220>
<223>  linker region

<400>  25

Thr Gly Gly Ser Gly Gly Ser Gly Gly Gly Gly Ser Gly Gly Ser Gly
1               5                   10                  15


Gly Met Ser



<210>  26
<211>  14
<212>  PRT
<213>  Artificial Sequence

<220>
<223>  cationization motif

<400>  26

Arg Pro Arg Pro Arg Pro Arg Pro Arg Pro Arg Pro Arg Pro
1               5                   10

<210>  27
<211>  210
<212>  PRT
<213>  Artificial Sequence

<220>
<223>  CMPX-383B

<400>  27

Met Gly His His His His His His His His His His Ser Ser Gly His
1               5                   10                  15

Ile Glu Gly Arg His Gly Pro Arg Pro Arg Pro Arg Pro Arg Pro Arg
            20                  25                  30

Pro Arg Pro Arg Pro Gly Gly Met Ser Thr Glu Gln Ile Gln Lys Arg
        35                  40                  45

Thr Ala Ala Ile Gln Lys Arg Ile Ala Ala Ile Gln Lys Arg Ile Tyr
    50                  55                  60

Ala Met Thr Ala Ser Gly Gly Ala Gly Ala Ala Gly Ala Gly Ala Gly
65                  70                  75                  80

Met Ser Ile Glu Glu Ile Thr Lys Gln Ile Ala Ala Ile Gln Leu Arg
                85                  90                  95

Ile Val Gly Asp Gln Val Gln Ile Ala Tyr Gln Thr Ala Ser Gly Ala
            100                 105                 110

Gly Ala Gly Ala Gly Gly Met Ser Thr Glu Glu Ile Gln Lys Gln Ile
        115                 120                 125

Ala Ala Ile Glu Thr Gln Ile Cys Lys Ile Glu Ala Ala Ile Glu Leu
    130                 135                 140

Lys Glu Ala Gly Ile Thr Ser Asp Phe Tyr Phe Glu Leu Ile Asn Lys
145                 150                 155                 160

Ala Lys Thr Cys Glu Ala Val Glu Ala Leu Lys Glu His Ile Leu Ala
                165                 170                 175

Ala His Thr Pro Arg Pro Arg Pro Arg Pro Arg Pro Arg Pro Arg Pro
            180                 185                 190

Arg Pro Gly Ser Gly Lys Pro Ile Pro Asn Pro Leu Leu Gly Leu Asp
        195                 200                 205

Ser Thr
    210

<210>  28
<211>  21
<212>  DNA
<213>  Artificial Sequence

<220>
<223>  forward primer MCL-1

<400>  28
gtcctctaca tggaagaact c                                                21

<210>  29
<211>  21
<212>  DNA
<213>  Artificial Sequence

<220>
<223>  reverse primer MCL-1

<400>  29
acacttgaag accataaacc a                                                21

<210>  30
<211>  19
<212>  DNA
<213>  Artificial Sequence

<220>
<223>  forward primer UBC

<400>  30
atttggtgcg cggttcttg                                                   19

<210>  31
<211>  21
<212>  DNA
<213>  Artificial Sequence

<220>
<223>  reverse primer UBC

<400>  31
tgccttgaca ttctcgatgg t                                                21

<210>  32
<211>  20
<212>  DNA
<213>  Artificial Sequence

<220>
<223>  forward primer TBP

<400>  32
cacgaaccac ggcactgatt                                                   20


<210>  33
<211>  22
<212>  DNA
<213>  Artificial Sequence

<220>
<223>  reverse primer TBP

<400>  33
ttttcttgct gccagtctgg ac                                                22


<210>  34
<211>  21
<212>  DNA
<213>  Artificial Sequence

<220>
<223>  forward primer HPRT1

<400>  34
tgacactggc aaaacaatgc a                                                 21


<210>  35
<211>  21
<212>  DNA
<213>  Artificial Sequence

<220>
<223>  reverse primer HPRT1

<400>  35
ggtccttttc accagcaagc t                                                 21


<210>  36
<211>  26
<212>  PRT
<213>  Artificial Sequence

<220>
<223>  HRS1

<400>  36

Thr Glu Gln Ile Gln Lys Arg Thr Ala Ala Ile Gln Lys Arg Ile Ala
1               5                   10                  15


Ala Ile Gln Lys Arg Ile Tyr Ala Met Thr
            20                  25


<210>  37
<211>  350
<212>  PRT
<213>  Homo sapiens

<400> 37

Met Phe Gly Leu Lys Arg Asn Ala Val Ile Gly Leu Asn Leu Tyr Cys
1               5                   10                  15

Gly Gly Ala Gly Leu Gly Ala Gly Ser Gly Gly Ala Thr Arg Pro Gly
            20                  25                  30

Gly Arg Leu Leu Ala Thr Glu Lys Glu Ala Ser Ala Arg Arg Glu Ile
        35                  40                  45

Gly Gly Gly Glu Ala Gly Ala Val Ile Gly Gly Ser Ala Gly Ala Ser
    50                  55                  60

Pro Pro Ser Thr Leu Thr Pro Asp Ser Arg Arg Val Ala Arg Pro Pro
65                  70                  75                  80

Pro Ile Gly Ala Glu Val Pro Asp Val Thr Ala Thr Pro Ala Arg Leu
                85                  90                  95

Leu Phe Phe Ala Pro Thr Arg Arg Ala Ala Pro Leu Glu Glu Met Glu
            100                 105                 110

Ala Pro Ala Ala Asp Ala Ile Met Ser Pro Glu Glu Glu Leu Asp Gly
        115                 120                 125

Tyr Glu Pro Glu Pro Leu Gly Lys Arg Pro Ala Val Leu Pro Leu Leu
    130                 135                 140

Glu Leu Val Gly Glu Ser Gly Asn Asn Thr Ser Thr Asp Gly Ser Leu
145                 150                 155                 160

Pro Ser Thr Pro Pro Pro Ala Glu Glu Glu Glu Asp Glu Leu Tyr Arg
                165                 170                 175

Gln Ser Leu Glu Ile Ile Ser Arg Tyr Leu Arg Glu Gln Ala Thr Gly
            180                 185                 190

Ala Lys Asp Thr Lys Pro Met Gly Arg Ser Gly Ala Thr Ser Arg Lys
        195                 200                 205

Ala Leu Glu Thr Leu Arg Arg Val Gly Asp Gly Val Gln Arg Asn His
    210                 215                 220

Glu Thr Ala Phe Gln Gly Met Leu Arg Lys Leu Asp Ile Lys Asn Glu
225                 230                 235                 240

Asp Asp Val Lys Ser Leu Ser Arg Val Met Ile His Val Phe Ser Asp
                245                 250                 255

Gly Val Thr Asn Trp Gly Arg Ile Val Thr Leu Ile Ser Phe Gly Ala
            260                 265                 270

Phe Val Ala Lys His Leu Lys Thr Ile Asn Gln Glu Ser Cys Ile Glu
        275                 280                 285

Pro Leu Ala Glu Ser Ile Thr Asp Val Leu Val Arg Thr Lys Arg Asp
    290                 295                 300

Trp Leu Val Lys Gln Arg Gly Trp Asp Gly Phe Val Glu Phe Phe His
305                 310                 315                 320

Val Glu Asp Leu Glu Gly Gly Ile Arg Asn Val Leu Leu Ala Phe Ala
                325                 330                 335

Gly Val Ala Gly Val Gly Ala Gly Leu Ala Tyr Leu Ile Arg
            340                 345                 350

<210>  38
<211>  7
<212>  PRT
<213>  Artificial Sequence

<220>
<223>  heptad motif


<220>
<221>  SITE
<222>  (1)..(1)
<223>  is an apolar or hydrophobic amino acid residue

<220>
<221>  SITE
<222>  (2)..(3)
<223>  is a polar or hydrophilic amino acid residue

<220>
<221>  SITE
<222>  (4)..(4)
<223>  is an apolar or hydrophobic amino acid residue

<220>
<221>  SITE
<222>  (5)..(7)
<223>  is a polar or hydrophilic amino acid residue

<400>  38

Xaa Xaa Xaa Xaa Xaa Xaa Xaa
1               5

<210>  39
<211>  7
<212>  PRT
<213>  Artificial Sequence

<220>
<223>  heptad motif


<220>
<221>  SITE
<222>  (1)..(1)
<223>  is an apolar or hydrophobic amino acid residue

<220>
<221>  SITE
<222>  (2)..(4)
<223>  is a polar or hydrophilic amino acid residue

<220>
<221>  SITE
<222>  (5)..(5)
<223>  is an apolar or hydrophobic amino acid residue

<220>
<221>  SITE
<222>  (6)..(7)
<223>  is a polar or hydrophilic amino acid residue

<400>  39

Xaa Xaa Xaa Xaa Xaa Xaa Xaa
1               5


<210>  40
<211>  155
<212>  PRT
<213>  Homo sapiens

<400>  40

Glu Leu Tyr Arg Gln Ser Leu Glu Ile Ile Ser Arg Tyr Leu Arg Glu
1               5                   10                  15


Gln Ala Thr Gly Ala Lys Asp Thr Lys Pro Met Gly Arg Ser Gly Ala
            20                  25                  30


Thr Ser Arg Lys Ala Leu Glu Thr Leu Arg Arg Val Gly Asp Gly Val
        35                  40                  45


Gln Arg Asn His Glu Thr Ala Phe Gln Gly Met Leu Arg Lys Leu Asp
    50                  55                  60


Ile Lys Asn Glu Asp Asp Val Lys Ser Leu Ser Arg Val Met Ile His
65                  70                  75                  80


Val Phe Ser Asp Gly Val Thr Asn Trp Gly Arg Ile Val Thr Leu Ile

85                  90                  95

Ser Phe Gly Ala Phe Val Ala Lys His Leu Lys Thr Ile Asn Gln Glu
            100                 105                 110

Ser Cys Ile Glu Pro Leu Ala Glu Ser Ile Thr Asp Val Leu Val Arg
        115                 120                 125

Thr Lys Arg Asp Trp Leu Val Lys Gln Arg Gly Trp Asp Gly Phe Val
    130                 135                 140

Glu Phe Phe His Val Glu Asp Leu Glu Gly Gly
145                 150                 155

<210>  41
<211>  26
<212>  PRT
<213>  Artificial Sequence

<220>
<223>  scaffold

<400>  41

Ile Glu Glu Ile Gln Lys Gln Ile Ala Ala Ile Gln Lys Gln Ile Ala
1               5                   10                  15

Ala Ile Gln Lys Gln Ile Tyr Ala Met Thr
            20                  25

<210>  42
<211>  26
<212>  PRT
<213>  Artificial Sequence

<220>
<223>  alpha-helix A of CMPX-152A

<400>  42

Ile Glu Gln Ile Gln Lys Arg Thr Ala Ala Ile Gln Lys Arg Ile Ala
1               5                   10                  15

Ala Ile Gln Lys Arg Ile Tyr Ala Met Thr
            20                  25

<210>  43
<211>  26
<212>  PRT
<213>  Artificial Sequence

<220>
<223>  alpha-helix B of CMPX-152A

<400>  43

Ile Glu Glu Ile Thr Lys Gln Ile Ala Ala Ile Gln Leu Arg Ile Val
1               5                   10                  15

Gly Asp Gln Val Gln Ile Ala Tyr Met Thr
            20                  25

## Description

### FIELD OF THE INVENTION

The present invention relates to MCL-1 binding molecules that have an extended serum half-life, which molecules are suitable for use in prophylactic or therapeutic applications.

### BACKGROUND OF THE INVENTION

Myeloid cell leukaemia 1 (MCL-1) is a member of the anti-apoptotic BCL-2 family, a group of intracellular anti-apoptotic proteins that contain up to four BCL-2 homology (BH) regions. All members of this family are characterized by their ability to bind and antagonize apoptosis-initiating proteins, such as the pore-forming pro-apoptotic BCL-2 family members BAK and BAX.

Under normal conditions, programmed cell death is counteracted by anti-apoptotic proteins, such as MCL-1 and BCL-2. However, upon cellular stress, the pro-apoptotic BH3-only proteins are upregulated and bind to pro-apoptotic BCL-2 family members by docking of the BH3-only domain into a hydrophobic groove at the surface of the BCL-2 family member. This interaction results in activation of BAK and BAX that will initiate the programmed cell death cascade by permeabilization of the mitochondrial outer membrane.

Many tumours prevent apoptosis by the upregulation of the anti-apoptotic protein MCL-1, which makes it an attractive candidate for anti-cancer therapy. Flavopiridol, a pan-cyclin dependent kinase inhibitor, and AZD-4573, a selective CDK9 inhibitor, have been developed to indirectly downregulate MCL-1. In addition, chemotherapeutics such as anthracyclines are known to repress MCL-1 transcription, but all are associated with considerable side-effects limiting their therapeutic potential. So far, none of the more than 20 compounds evaluated in preclinical studies or clinical trials have resulted in the approval of an MCL-1 targeted therapy, despite its clear correlation with therapy resistance in various cancers.

Monoclonal antibodies are currently at the forefront of clinical treatments for a wide variety of cancers. Although therapeutic monoclonal antibodies are endowed with many favourable characteristics that are important for their use as therapeutic agents, such as long serum half-life, high specificity, and immunological effector functions, they are also critically limited by low tissue penetration and inability to directly address intracellular drug targets, such as MCL-1. Additionally, they impose high production or formulation costs due to low stability and the need for essential post-translational modifications, and harbour complexities in drug administration and patient care.

To overcome some of these impediments, alternative approaches emerged, including engineered antibody fragments, as well as non-antibody protein scaffolds. Interest in non-Ig protein scaffolds has been particularly intense due to the ease of screening and selection of high-affinity binders, exquisite stability, scalability, and low cost of production. However, despite successes in preclinical studies, the progression of non-antibody scaffolds to clinical development is often hampered by the fast clearance of such small-sized protein scaffolds.

There thus remains a need in the art for improved MCL-1 targeting agents.

### SUMMARY OF THE INVENTION

Present inventors have designed new polypeptides comprising an Alphabody structure which can combine high affinity to a target with extended half-life. In addition the inventors have found ways to combine these properties with the ability to access an intracellular target.

More particularly, the present inventors designed new polypeptides comprising an Alphabody structure with the ability to bind and inhibit MCL-1 with high affinity and having an extended half-life *in vivo.*

More particularly, present inventors found that engrafting key binding determinants inspired by the interaction of MCL-1with its inhibitory BH3 helix and other peptides, onto the several possible variable positions presented by the three-helix coiled-coiled fold of the Alphabody scaffold resulted in an Alphabody showing ultra-high affinity in the low pM range (e.g. KD = 8 pM) for MCL-1. Such tight binding is important for on-target affinity in MCL-1 dependent cell lines, and is more than 20-fold tighter than the reported affinity of S63845, the most promising MCL-1 inhibitor to date (KD of S63845: 190 pM).The present inventors engineered a serum half-life extension technology that is added to the MCL-1 binding Alphabody without interfering with MCL-1 binding and/or cell penetration. The key challenge was to achieve extension of the serum half-life of the MCL-1 binding Alphabodies, which is typically enhanced by increasing the mass of the protein, while maintaining a low molecular weight to preserve efficient cell-penetration. Present inventors found that this dichotomy could be resolved by grafting, rather than adding, the smallest albumin-binding helices of *F*. *magna* protein G onto the MCL-1binding Alphabody, resulting in a drastic extension of the serum half-life of the Alphabody.

In addition, present inventors also designed and successfully implemented a cellular uptake sequence motif that can enable efficient translocation of the MCL-1 binding Alphabody across the cell membrane to reach intracellular MCL-1, without loss of binding specificity.

Moreover, present inventors have demonstrated that the polypeptides comprising a MCL-1 binding Alphabody structure as taught herein can alleviate tumour burden in xenograft experiments based on multiple myeloma cell lines as well as primary T-ALL cells from patients.

Accordingly, an aspect provides a polypeptide capable of specifically binding myeloid cell leukaemia 1 (MCL-1) comprising at least one Alphabody structure having the general formula
HRS1-L1-HRS2-L2-HRS3,
- wherein each of HRS1, HRS2 and HRS3 is independently a heptad repeat sequence (HRS) comprising 2 to 7 consecutive but not necessarily identical heptad repeat units,
- wherein said heptad repeat units are 7-residue (poly)peptide fragments represented as 'abcdefg' or 'defgabc', wherein the symbols 'a' to 'g' denote conventional heptad positions,
- wherein at least 50% of all heptad a- and d-positions are occupied by isoleucine residues,
- wherein each HRS starts and ends with an aliphatic or aromatic amino acid residue located at a heptad a-position or d-position,
- wherein each of L1 and L2 are independently a linker fragment, which covalently connect HRS1 to HRS2 and HRS2 to HRS3, respectively,
characterized in that
- HRS2 comprises a MCL-1 binding region comprising a sequence LRXVGDXV (SEQ ID NO: 1), wherein X can be any amino acid;
- the polypeptide comprises an albumin binding region conjugated to the C-terminus of HRS3, wherein said albumin binding region comprises a sequence SDFYFXXINKA (SEQ ID NO: 2) and a sequence TXEXVXALKXXILXAH (SEQ ID NO: 3), wherein X can be any amino acid;
- HRS1, HRS2 and HRS3 form a triple-stranded, anti-parallel, alpha-helical coiled coil; and
- HRS 1, HRS2, HRS3 and the albumin binding region together form a five-stranded alpha-helical bundle.

In particular embodiments, the albumin binding region comprises a sequence SDFYFXXINKAKTXEXVXALKXXILXAH (SEQ ID NO: 4), preferably a sequence SDFYFXXINKAKTCEAVXALKXXILXAH (SEQ ID NO: 5), wherein X can be any amino acid.

In particular embodiments, the polypeptide comprises a peptide for facilitating cellular entry of the polypeptide, wherein said peptide comprises a sequence (RP)ₙ, wherein n is an integer from 6 to 9.

In particular embodiments, said peptide for facilitating cellular entry of the polypeptide is conjugated to the N- and/or C-terminus of the polypeptide as defined herein.

A further aspect provides a nucleic acid sequence encoding the polypeptide as taught herein.

A further aspect provides a pharmaceutical composition comprising the polypeptide as taught herein, and a pharmaceutically acceptable carrier.

A further aspect provides the polypeptide as taught herein or the pharmaceutical composition as taught herein for use as a medicament.

A further aspect provides the polypeptide as taught herein, or the pharmaceutical composition as taught herein for use in the treatment of an MCL-1 mediated disease or disorder, preferably a neoplastic disease or an autoimmune disease.

These and further aspects and preferred embodiments of the invention are described in the following sections and in the appended claims. The subject-matter of the appended claims is hereby specifically incorporated in this specification.

### DESCRIPTION OF THE DRAWINGS

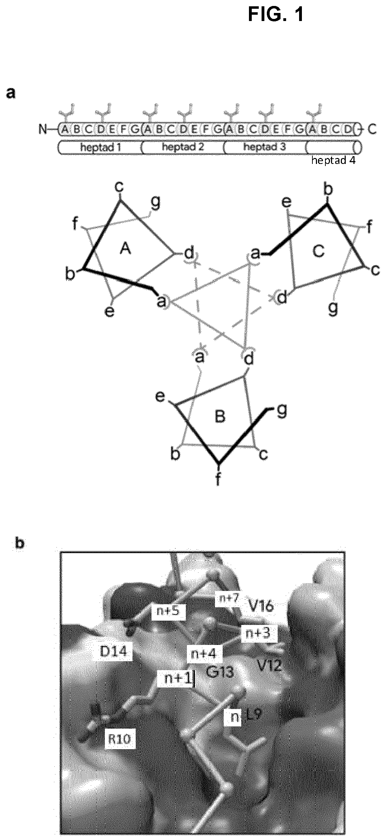

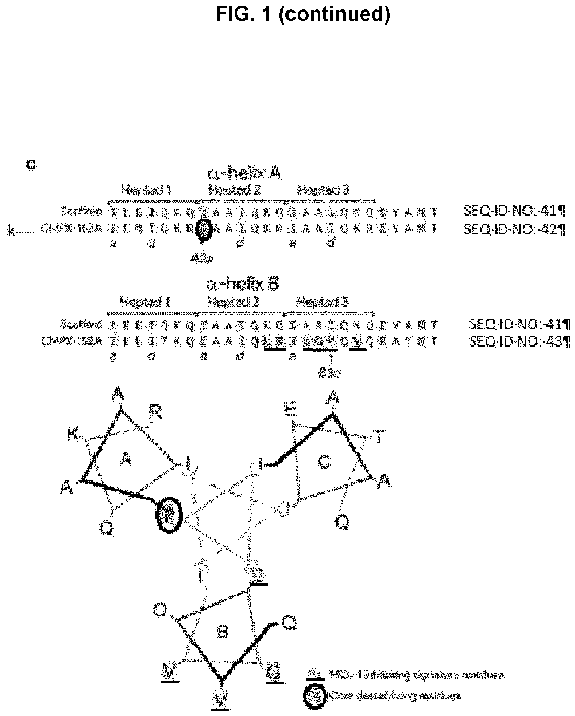

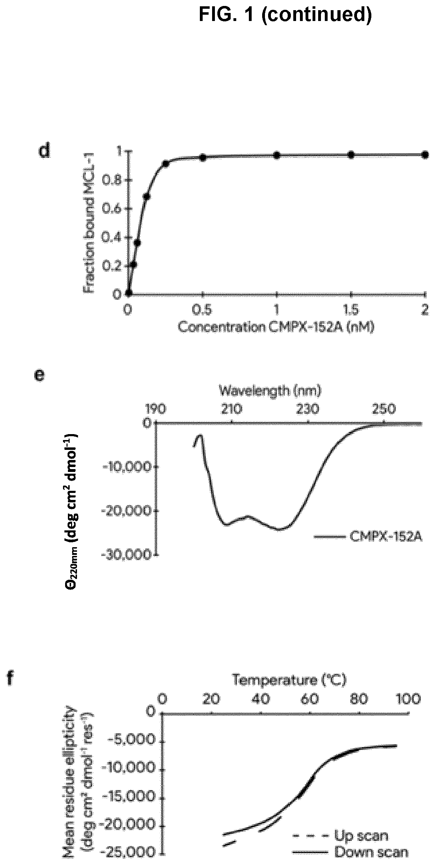

**FIG. 1** Grafting of the MCL-1 inhibiting motif onto the Alphabody scaffold results in a high-affinity MCL-1 binder. **a.** Overview of the Alphabody protein scaffold. Alphabodies are 3-helical coiled-coils consisting of, for example, 4 heptad repeat units (or 'heptads') each. N- or C-terminal heptads may start or end at an a- or d-position. Hydrophobic core interactions between isoleucines at positions a and d of each heptad stabilize the fold and result in an extremely high thermal stability and other favourable physicochemical parameters (J. Desmet, et al., Structural basis of IL-23 antagonism by an Alphabody protein scaffold, Nat. Commun. 5, 5237 (2014)). Upper: linear representation of one helix. Lower: cross-sectional helical wheel representation of the interaction between core positions in the coiled-coil. Positions within one triangle belong to the same core layer. Figure panel adapted from (J. Desmet, et al., Structural basis of IL-23 antagonism by an Alphabody protein scaffold, Nat. Commun. 5, 5237 (2014)). **b.** Six residues describe the MCL-1 inhibition binding motif to the BH3-binding groove. The 6 residues (stick representation) were delineated based on available complexes of MCL-1 in complex with an inhibitory peptide. The backbone chain of this inhibitory helix, docked into the BH3-binding groove of MCL-1, is shown in sticks representation, Cα atoms are shown as spheres. MCL-1 is shown in surface representation and coloured according to the Eisenberg hydrophobicity scale (darker grey is more hydrophobic). Figure generated using PBD 3MK8. **c.** Incorporation of the MCL-1 inhibitory footprint onto the Alphabody scaffold required a polar core layer. Upper: 6 MCL-1 key-binding residues (grey background and underlined) were grafted onto helix B of CMPX-152A, which inserted Aspₙ₊₅ (grey background and light grey font colour) into the all-Ile Alphabody core on position d of the 3rd heptad of α-helix B (position B3d, indicated). To maintain structural stability of CMPX-152A, the Ile at position a of the 2^{nd} heptad of α-helix A (position A2a, indicated) was mutated into a threonine (grey background and circled). Lower: schematic cross-section of CMPX-152A showing the Asp_{B3d}-containing core layer. In silico modelling revealed that the most stable conformation of an Alphabody would cluster Thr_{A2a} and Asp_{B3d} into the same core layer of interacting core residues (grey triangle in full lines). **d.** Determination by solution ELISA of the affinity of Alphabody CMPX-152A for MCL-1. **e.** Secondary-structure characterization in CMPX-152A by circular-dichroism revealing an all alpha-helical content. **f.** TM (melting temperature) determination of CMPX-152A by circular dichroism thermal denaturation experiment.

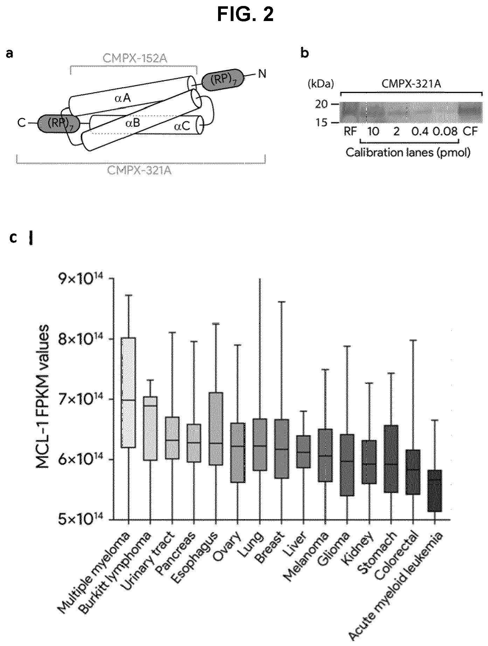

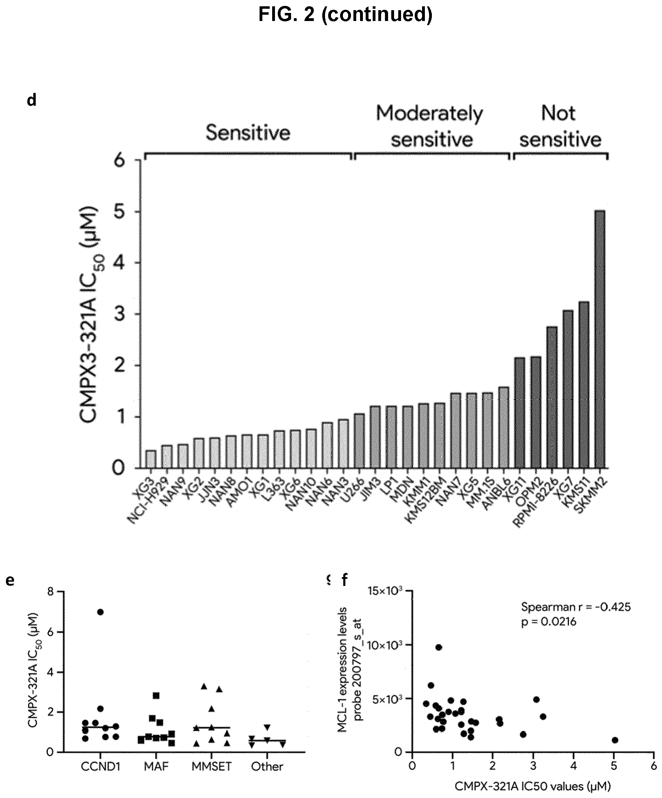

**FIG. 2** Cell penetrating Alphabody (CPAB)-formatted CMPX-321A Alphabody efficiently enters multiple myeloma cell lines to elicit MCL-1 inhibition **a.** Addition of an N- and C-terminal arginine-proline ([RP]₇) tag allows bilayer phospholipid crossing of CMPX-321A, the CPAB-formatted successor of CMPX-152A. **b.** Western blot of the rest fraction (R.F.) and cytosolic fraction (C.F.) of NCI-H929 cells after 2 h incubation with 2 µM of the cell-penetrating Alphabody CMPX-321A. **c.** RNA expression data shows that MCL-1 is substantially more expressed in multiple myeloma (MM) cells compared to other cancer cell types, indicating that this cancer cell type might be MCL-1 dependent. **d.** The cell killing potential of CMPX-321A was evaluated via a panel of 33 MM cell lines. Thirteen MM cell lines were shown to be very sensitive towards MCL-1 inhibition (IC₅₀ < 1µM), 10 cell lines were moderately sensitive (IC50 = 1 - 2µM), and 6 cell lines not sensitive to unresponsive (IC50 > 2µM). **e.** The cell killing potency of CMPX-321A cannot be predicted based on the classical molecular signature of MM cell lines (cyclin D1 (CCND1), MAF, multiple myeloma SET domain (MMSET) and others). **f**. CMPX-321A response was analysed in function of MCL-1 gene expression. For CMPX-321A, a statistically significant correlation between the CPAB response and MCL-1 gene expression of MM cell lines could be detected (p = 0.0216, Spearman rank correlation), indicating that the IC50 of the CMPX-321A treatment is indeed inversely correlated with MCL-1 gene expression.

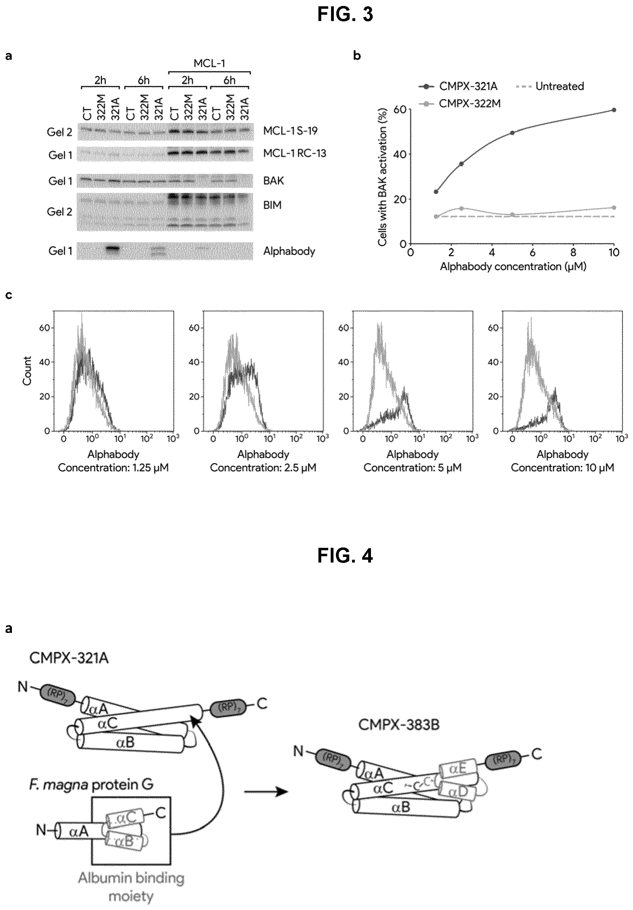

**FIG. 3** Cell-penetrating Alphabody CMPX-321A induces apoptosis in NCI-H929 cells by targeting MCL-1. **a.** Western blot analysis of MCL-1-immunoprecipitated fractions after treatment with 1 µM Alphabody CMPX-321A shows a disruption of MCL-1: BAK and MCL-1 :BIM complexes after 2h and 6h respectively. No disruption could be detected when using the non-MCL-1 binding CPAB-formatted Alphabody CMPX-322M, or in absence of treatment (CT). **b.** Percentage of BCL-2 homologous antagonist killer (BAK)-activated positive NCI-H929 cells following CPAB treatment with anti-MCL-1 CPAB CMPX-321A (dark grey) and control CPAB CMPX-322M (light grey). CMPX-321A induces a dose-dependent BAK activation. The control CPAB is not inducing BAK activation and is comparable to non-treated cells **c.** Flow cytometric histograms showing overlay plots of NCI-H929 cells treated with control CPAB CMPX-322M (light grey) and anti-MCL-1 CPAB CMPX-321A (dark grey) that have been stained with an antibody specifically recognizing activated BAK.

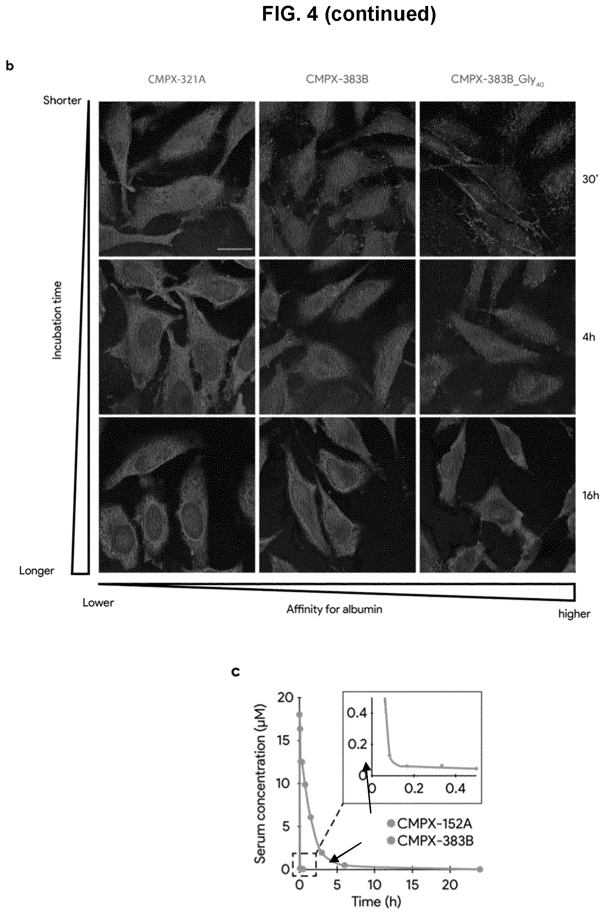

**FIG. 4** Engineering of an albumin binding site in CMPX-321A to increase its serum half-life. **a.** By grafting only the short albumin binding helices of F. magna Protein G (albumin binding moiety in box) on α-helix C of CMPX-321A, the resulting CPAB CMPX-383B is capable of binding albumin at the expense of an only 3.6 kDa increase in molecular weight. **b**. Confocal microscopy experiments on HeLa cells in presence of 10 µM MSA show that substitution of Gly40 by alanine (right and middle column, respectively) allows a faster cellular uptake. After the indicated incubation time, cells were washed and probed for the presence of the Alphabody (light grey). The resulting immunofluorescence image was overlaid with a Ruby stain for the nuclei. **c.** Alphabody concentrations of CMPX-321A and the serum half-life extended Alphabody CMPX-383B in the sera of mice, treated with a single intravenously administered bolus injection of 20 mg/kg.

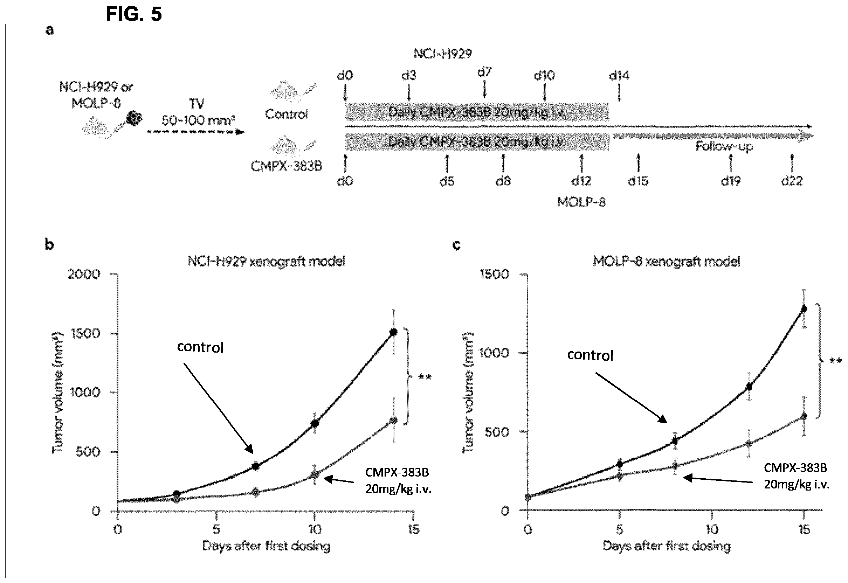

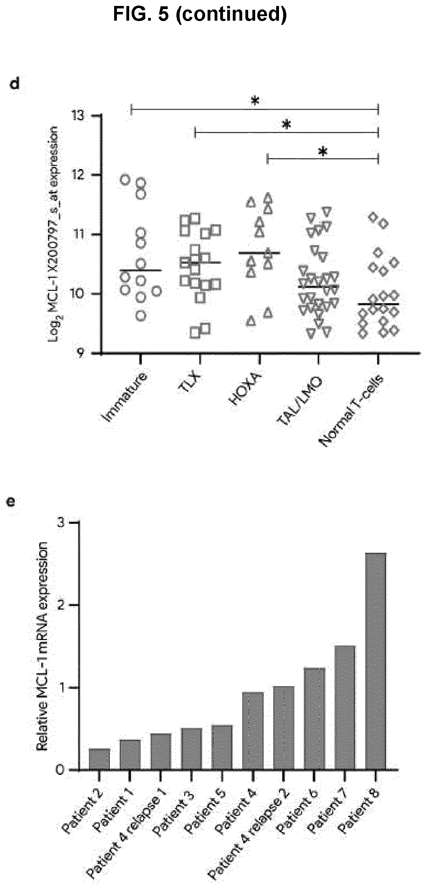

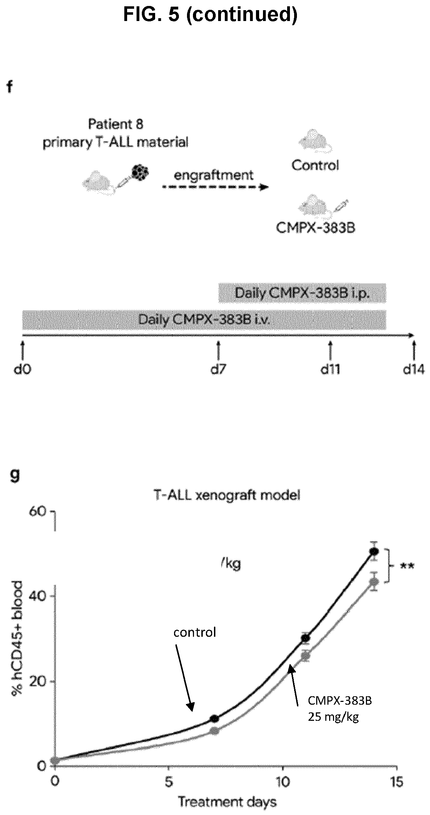

**FIG. 5** In vivo anti-tumour effect of CMPX-383B in an MM and T-cell acute lymphoblastic leukaemia (T-ALL) xenograft model. **a.** Therapy schedule followed for the treatment of both MM xenograft models. **b.** Treatment of NCI-H929 xenograft mice for 14 days with CMPX-383B significantly decreases tumour volume growth in CMPX-383B treated mice, compared to vehicle (p = 0.002). **c.** Treatment of the MOLP-8 xenograft model with CMPX-383B results in a significant decrease in tumour volume growth during the treatment period (p = 0.007). **d.** A micro-array gene expression profiling on a panel of 64 primary T-ALL patients shows that MCL-1 was significantly higher expressed in most T-ALL subgroups compared to normal T-cells (Immature: p = 0.0224 ; TLX: p = 0.0320; homeobox A (HOXA): p = 0.0205) **e.** MCL-1 mRNA expression levels in a panel of primary T-ALL patients, ranked according to their MCL-1 tumour expression levels. Primary material of patient 8, which showed the highest MCL-1 expression level, was used to establish a patient-derived xenograft (PDX) for the in vivo treatment with CMPX-383B. **f.** Primary T-ALL cells of patient 8 were injected in immunodeficient mice. Immediately after engraftment, CMPX-383B treatment was initiated according to the schedule depicted here. **g.** Treatment of a T-ALL patient-established xenograft significantly delays the amount of hCD45+ leukemic cells in the blood (p = 0.006).

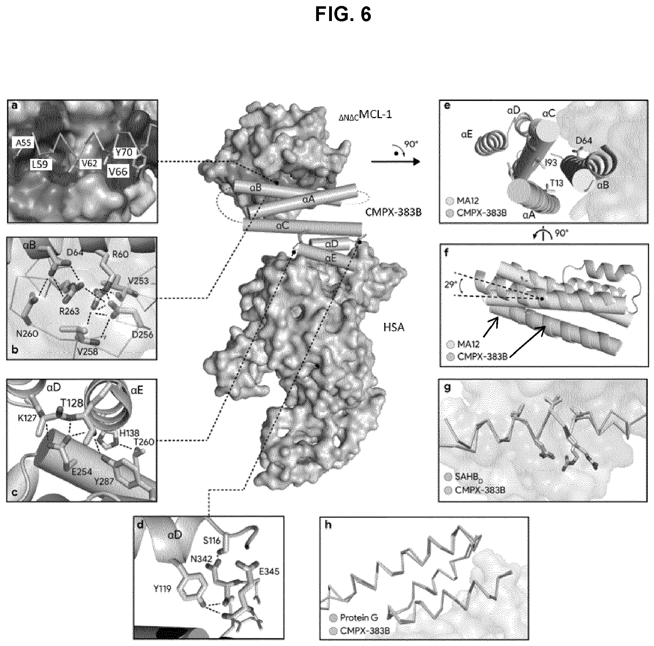

**FIG. 6** Structural validation of the applied engineering strategies. Middle panel: structural superposition of crystal structures featuring CMPX-383B in complex with either _{ΔNΔC}MCL1, or human serum albumin (HSA). Alphabody CMPX-383B is shown in cartoon representation. The linker regions between α-helix A and B, and between α-helix B and C are represented as dotted lines. The cysteine disulfide bridge that covalently links α-helix E to C is shown in stick representation. _{ΔNΔC}MCL1 and HSA are shown in surface representation. The BH3 binding groove of _{ΔNΔC}MCL1 is represented by a darker shade of grey. **a.** Detailed view of the hydrophobic interface between α-helix B of CMPX-383B and the BH3 binding groove of _{ΔNΔC}MCL1. Helix B of CMPX-383B is shown as ribbon diagram, _{ΔNΔC}MCL1 is shown in surface representation and coloured according to the Eisenberg hydrophobicity scale (a darker shade of grey is more hydrophobic). **b.** Detailed view of the electrostatic interactions around Asp₆₄ and Arg₆₀ of CMPX-383B at the BH3 binding groove of _{ΔNΔC}MCL1. **c.** and **d.** provide detailed views on how CMPX-383B engages HSA using predominantly electrostatic interactions at α-helix D, the αD-αE loop and α-helix E. **e.** and **f.** display a structural superposition of CMPX-383B (ribbon representation) and the canonical IL-23 binding Alphabody MA12 (cylindrical helix presentation, generated using PBD code 5MJ3), highlighting the distinct arching of α-helix B of CMPX-383B allowing a full occupancy of the BH3 binding groove of _{ΔNΔC}MCL1 (surface representation). **g.** Structural superposition of the inhibitory MCL-1 helix SAHB_{D} (PDB code 3MK8) with α-helix B of CMPX-383B, both when in complex with MCL-1 (surface representation). **h.** Structural superimposition of the albumin-binding moiety of F. magna Protein G (generated using PDB code 1TF0) and α-helix C, D and E of CMPX-383B, both bound to HSA (surface representation).

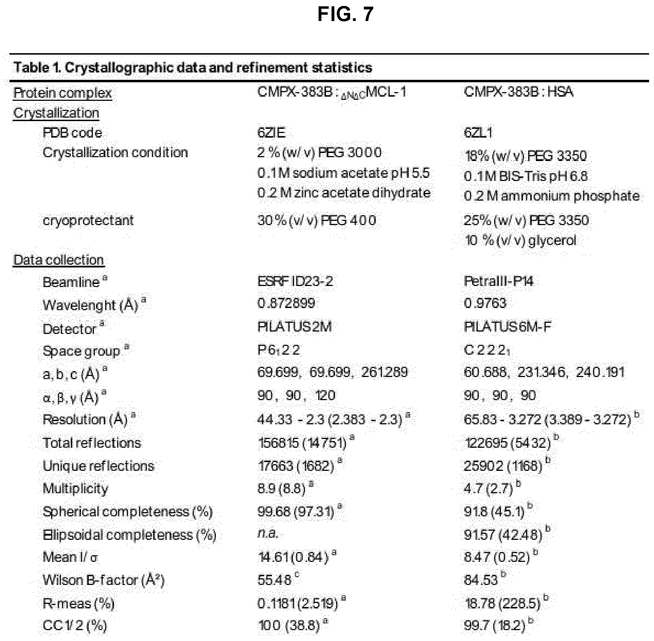

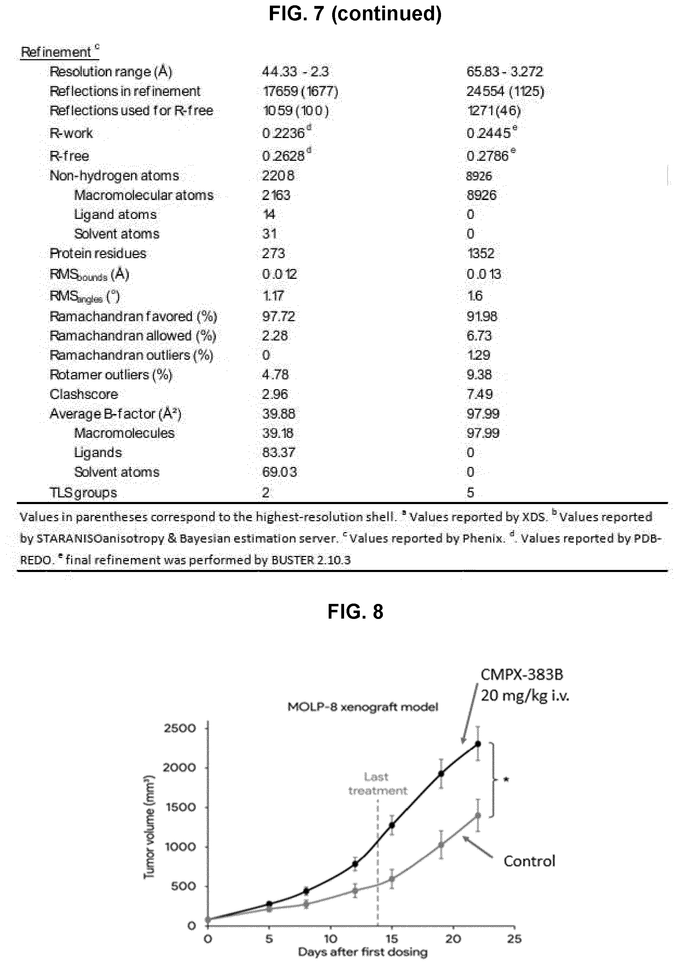

**FIG. 7** Table 1. Crystallographic data and refinement statistics
**FIG. 8** CMPX-383B-mediated reduction of tumour progression in a MOLP-8 xenograft model remains significant after treatment. After daily injections with CMPX-383B for a period of 14 days, MOLP-8 xenograft mice were monitored for an extended period of 7 days without therapy. CMPX-383B administration significantly reduced tumour growth over the whole data range (day 0 to day 22) (p = 0.027). The CMPX-383B-induced delay in tumour progression is only nearly significant for the period without treatment (day 15 to day 22) (p = 0.051), indicating that the significance of the overall delay is rooted at the delay in tumour growth during therapy (day 0 to day 15) (p = 0.007).

### DETAILED DESCRIPTION OF THE INVENTION

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms also encompass "consisting of' and "consisting essentially of', which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of ± 10% or less, preferably ± 5% or less, more preferably ± 1% or less, and still more preferably ± 0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

As used herein, an 'Alphabody' or 'Alphabodies' can generally be defined as sequences of amino acids which are single-chain, triple-stranded, predominantly alpha-helical, coiled coil amino acid sequences. More particularly, an Alphabody structure as used in the context of the present invention can be defined as an amino acid sequences having the general formula HRS1-L1-HRS2-L2-HRS3, wherein
- each of HRS1, HRS2 and HRS3 is independently a heptad repeat sequence (HRS) comprising or consisting of 2 to 7 consecutive but not necessarily identical heptad repeat units, at least 50% of all heptad a- and d-positions are occupied by isoleucine residues, each HRS starts and ends with an aliphatic or aromatic amino acid residue located at a heptad a-position or d-position;
- each of L1 and L2 are independently a linker fragment, as further defined hereinafter, which covalently connect HRS1 to HRS2 and HRS2 to HRS3, respectively.
In the Alphabody or Alphabody structure as defined above, HRS1, HRS2 and HRS3 will together form a triple-stranded, alpha-helical, coiled coil structure.

As used herein, an 'antiparallel Alphabody' refers to an Alphabody as defined above, further characterized in that the alpha-helices of the triple-stranded, alpha-helical, coiled coil structure together form an antiparallel coiled coil structure, i.e., a coiled coil wherein two alpha-helices are parallel and the third alpha-helix is antiparallel with respect to these two helices.

As will become clear from the further description herein, the application envisages polypeptides comprising an amino acid sequence with the general formula HRS 1-L1-HRS2-L2-HRS3, but which in certain particular embodiments may comprise additional residues, moieties and/or groups which are covalently linked, more particularly N- and/or C-terminally covalently linked, to a basic Alphabody sequence structure having the formula HRS1-L1-HRS2-L2-HRS3. Thus reference is made herein generally to '(Alphabody) polypeptides' which comprise or consist of an Alphabody as defined above, which may be covalently linked to additional sequences. The binding features described for an Alphabody herein can generally also be applied to polypeptides comprising said Alphabody.
The terms 'heptad', 'heptad unit' or 'heptad repeat unit' are used interchangeably herein and shall herein have the meaning of a 7-residue (poly)peptide motif that is repeated two or more times within each heptad repeat sequence of an Alphabody structure, and is represented as 'abcdefg' or 'defgabc', wherein the symbols 'a' to 'g' denote conventional heptad positions. As understood by the skilled person this implies that the consecutive heptad units within a repeat need not contain the same amino acids but will contain the same type of amino acid (hydrophobic vs. polar, as detailed below) at the same position. Conventional heptad positions are assigned to specific amino acid residues within a heptad, a heptad unit, or a heptad repeat unit, present in an Alphabody structure, for example, by using specialized software such as the COILS method of Lupas et al. (Science 1991, 252:1162-1164; https://embnet.vitalit.ch/software/COILS_form.html). However, it is noted that the heptads or heptad units as present in the Alphabody structure are not strictly limited to the above-cited representations (i.e. 'abcdefg' or 'defgabc') as will become clear from the further description herein and in their broadest sense constitute a 7-residue (poly)peptide fragment per se, comprising at least assignable heptad positions a and d.
The terms 'heptad a-positions', 'heptad b-positions', 'heptad c-positions', 'heptad d-positions', 'heptad e-positions', 'heptad f-positions' and 'heptad g-positions' refer respectively to the conventional 'a', 'b', 'c', 'd', 'e', 'f and 'g' amino acid positions in a heptad, heptad repeat or heptad repeat unit.

A heptad motif (as defined herein) of the type 'abcdefg' is typically represented as 'HPPHPPP' (SEQ ID NO: 38, whereas a 'heptad motif of the type 'defgabc' is typically represented as 'HPPPHPP', wherein the symbol 'H' denotes an apolar or hydrophobic amino acid residue and the symbol 'P' denotes a polar or hydrophilic amino acid residue. Typical hydrophobic residues located at a- or d-positions include aliphatic (e.g., leucine, isoleucine, valine, methionine) or aromatic (e.g., phenylalanine) amino acid residues. Heptads within coiled coil sequences do not always comply with the ideal pattern of hydrophobic and polar residues, as polar residues are occasionally located at 'H' positions and hydrophobic residues at 'P' positions. Thus, the patterns 'HPPHPPP' (SEQ ID NO: 38) and 'HPPPHPP' (SEQ ID NO: 39) are to be considered as ideal patterns or characteristic reference motifs.

A 'heptad repeat sequence' ('HRS') as used herein shall have the meaning of an amino acid sequence or sequence fragment comprising or consisting of n consecutive heptads, where n is a number equal to or greater than 2.

A heptad repeat sequence (HRS) can thus generally be represented by (abcdefg)ₙ or (defgabc)ₙ in notations referring to conventional heptad positions, or by (HPPHPPP)ₙ or (HPPPHPP)ₙ in notations referring to the heptad motifs, with the proviso that a) the amino acids at positions a-g or H and P need not be identical amino acids in the different heptads, b) not all amino acid residues in a HRS should strictly follow the ideal pattern of hydrophobic and polar residues, and c) the HRS may end with an incomplete or partial heptad motif. With regard to the latter, in particular embodiments, the HRS may contain an additional sequence "a", "ab", "abc", "abed", "abcde", or "abcdef" following C-terminally of the (abcdefg)ₙ sequence. However, in particular embodiments of the Alphabody structure as envisaged herein, a 'heptad repeat sequence' ('HRS') is an amino acid sequence or sequence fragment comprising n consecutive (but not necessarily identical) heptads generally represented by abcdefg or defgabc, where n is a number equal to or greater than 2, wherein at least 50% of all heptad a- and d-positions are occupied by isoleucine residues, each HRS starting with a full heptad sequence abcdefg or defgabc, and ending with a partial heptad sequence abcd or defga, such that each HRS starts and ends with an aliphatic or aromatic amino acid residue located at either a heptad a- or d-position.

In order to identify heptad repeat sequences, and/or their boundaries, these heptad repeat sequences comprising amino acids or amino acid sequences that deviate from the consensus motif, and if only amino acid sequence information is at hand, then the COILS method of Lupas et al. (Science 1991, 252:1162-1164) is a suitable method for the determination or prediction of heptad repeat sequences and their boundaries, as well as for the assignment of heptad positions. Furthermore, the heptad repeat sequences can be resolved based on knowledge at a higher level than the primary structure (i.e., the amino acid sequence). Indeed, heptad repeat sequences can be identified and delineated on the basis of secondary structural information (i.e. alpha-helicity) or on the basis of tertiary structural (i.e., protein folding) information. A typical characteristic of a putative HRS is an alpha-helical structure. Another (strong) criterion is the implication of a sequence or fragment in a coiled coil structure. Any sequence or fragment that is known to form a regular coiled coil structure, i.e., without stutters or stammers as described in Brown et al. Proteins 1996, 26:134-145, is herein considered a HRS. Also and more particularly, the identification of HRS fragments can be based on high-resolution 3-D structural information (X-ray or NMR structures). Finally, in particular embodiments, the boundaries to an HRS fragment may be defined as the first a- or d-position at which a standard hydrophobic amino acid residue (selected from the group valine, isoleucine, leucine, methionine, phenylalanine, tyrosine or tryptophan) is located. In particular embodiments, the boundaries to an HRS fragment can be defined by the presence of an isoleucine amino acid residue.

In the context of the single-chain structure of the Alphabodies (as defined herein) the terms 'linker', 'linker fragment' or 'linker sequence' are used interchangeably herein and refer to an amino acid sequence fragment that is part of the contiguous amino acid sequence of a single-chain Alphabody, and which covalently interconnect the HRS sequences of that Alphabody structure.

The linkers within a single-chain structure of the Alphabodies (as defined herein) thus interconnect the HRS sequences, and more particularly the first to the second HRS, and the second to the third HRS in an Alphabody structure. Each linker sequence in an Alphabody structure commences with the residue following the last heptad residue of the preceding HRS and ends with the residue preceding the first heptad residue of the next HRS. Connections between HRS fragments via disulfide bridges or chemical cross-linking or, in general, through any means of inter-chain linkage (as opposed to intra-chain linkage), are explicitly excluded from the definition of a linker fragment (at least, in the context of an Alphabody) because such would be in contradiction with the definition of a single-chain Alphabody. A linker fragment in an Alphabody structure is preferably flexible in conformation to ensure relaxed (unhindered) association of the three heptad repeat sequences as an alpha-helical coiled coil structure. Further in the context of an Alphabody, 'L1' shall denote the linker fragment one, i.e., the linker between HRS1 and HRS2, whereas 'L2' shall denote the linker fragment two, i.e., the linker between HRS2 and HRS3. Suitable linkers for use in the polypeptides envisaged herein will be clear to the skilled person, and may generally be any linker used in the art to link amino acid sequences, as long as the linkers are structurally flexible, in the sense that they do not affect the characteristic three dimensional coiled coil structure of the Alphabody. The two linkers L1 and L2 in a particular Alphabody structure may be the same or may be different. Based on the further disclosure herein, the skilled person will be able to determine the optimal linkers, optionally after performing a limited number of routine experiments. In particular embodiments, the linkers L1 and L2 are amino acid sequences consisting of at least 4, in particular at least 8, more particularly at least 12 amino acid residues, with a non-critical upper limit chosen for reasons of convenience being about 30 amino acid residues. In a particular, non-limiting embodiment, preferably at least 50% of the amino acid residues of a linker sequence are selected from the group proline, glycine, and serine. In further non-limiting embodiments, preferably at least 60%, such as at least 70%, such as for example 80% and more particularly 90% of the amino acid residues of a linker sequence are selected from the group proline, glycine, and serine. In other particular embodiments, the linker sequences essentially consist of polar amino acid residues; in such particular embodiments, preferably at least 50%, such as at least 60%, such as for example 70% or 80% and more particularly 90% or up to 100% of the amino acid residues of a linker sequence are selected from the group consisting of glycine, serine, threonine, alanine, proline, histidine, asparagine, aspartic acid, glutamine, glutamic acid, lysine and arginine.

In the present application, reference to a 'coiled coil' or 'coiled coil structure' shall be used interchangeably herein and will be clear to the person skilled in the art based on the common general knowledge and the description and further references cited herein. Particular reference in this regard is made to review papers concerning coiled coil structures, such as for example, Cohen and Parry Proteins 1990, 7:1-15; Kohn and Hodges Trends Biotechnol 1998, 16:379-389; Schneider et al Fold Des 1998, 3:R29-R40; Harbury et al. Science 1998, 282:1462-1467; Mason and Arndt ChemBioChem 2004, 5:170-176; Lupas and Gruber Adv Protein Chem 2005, 70:37-78; Woolfson Adv Protein Chem 2005, 70:79-112; Parry et al. J Struct Biol 2008, 163:258-269; McFarlane et al. EurJ Pharmacol 2009:625:101-107.

A 'solvent-oriented' or 'solvent-exposed' region of an alpha-helix of an Alphabody structure shall herein have the meaning of that part on an Alphabody structure which is directly exposed or which comes directly into contact with the solvent, environment, surroundings or milieu in which it is present. The solvent-oriented region is largely formed by b-, c- and f-residues. There are three such regions per single-chain Alphabody, i.e., one in each alpha-helix. Any part of such solvent-oriented region is also considered a solvent-oriented region. For example, a sub-region composed of the b-, c- and f-residues from three consecutive heptads in an Alphabody alpha-helix will also form a solvent-oriented surface region.

The term 'groove of an Alphabody' shall herein have the meaning of that part on an Alphabody of a polypeptide as envisaged herein which corresponds to the concave, groove-like local shape, which is formed by any pair of spatially adjacent alpha-helices within said Alphabody. Residues implicated in the formation of (the surface of) a groove between two adjacent alpha-helices in an Alphabody are generally located at heptad e- and g-positions, but some of the more exposed b- and c-positions as well as some of the largely buried core a- and d-positions may also contribute to a groove surface; such will essentially depend on the size of the amino acid side-chains placed at these positions. If the said spatially adjacent alpha-helices run parallel, then one half of the groove is formed by b- and e-residues from a first helix and the second half by c- and g-residues of the second helix. If the said spatially adjacent alpha-helices are antiparallel, then there exist two possibilities. In a first possibility, both halves of the groove are formed by b- and e-residues. In the second possibility, both halves of the groove are formed by c- and g-residues. The three types of possible grooves are herein denoted by their primary groove-forming (e- and g-) residues: if the helices are parallel, then the groove is referred to as an e/g-groove; if the helices are antiparallel, then the groove is referred to as either an e/e-groove or a g/g-groove.

As used herein, amino acid residues will be indicated either by their full name or according to the standard three-letter or one-letter amino acid code.

As used herein, the term 'homology' denotes structural similarity between two macromolecules, particularly between two polypeptides or polynucleotides, from same or different taxons, wherein said similarity is due to shared ancestry. Hence, the term 'homologues' denotes so-related macromolecules having primary and optionally secondary and tertiary structural similarity. For comparing two or more nucleotide sequences, the '(percentage of) sequence identity' between a first nucleotide sequence and a second nucleotide sequence may be calculated using methods known by the person skilled in the art, e.g. by dividing the number of nucleotides in the first nucleotide sequence that are identical to the nucleotides at the corresponding positions in the second nucleotide sequence by the total number of nucleotides in the first nucleotide sequence and multiplying by 100% or by using a known computer algorithm for sequence alignment such as NCBI Blast. In determining the degree of sequence identity between two Alphabodies, the skilled person may take into account so-called 'conservative' amino acid substitutions, which can generally be described as amino acid substitutions in which an amino acid residue is replaced with another amino acid residue of similar chemical structure and which has little or essentially no influence on the function, activity or other biological properties of the polypeptide. Possible conservative amino acid substitutions will be clear to the person skilled in the art. Alphabodies and nucleic acid sequences are said to be 'exactly the same' if they have 100% sequence identity over their entire length.

A polypeptide (or the Alphabody structure comprised therein) is said to 'specifically bind to' a particular target when that polypeptide has affinity for, specificity for, and/or is specifically directed against that target (i.e., against at least one part or fragment thereof).

The 'specificity' of an anti- MCL-1 polypeptide as used herein can be determined based on affinity and/or avidity. The 'affinity' of a polypeptide is represented by the equilibrium constant for the dissociation of the polypeptide and MCL-1. The lower the KD value, the stronger the binding strength between the polypeptide and MCL-1. Alternatively, the affinity can also be expressed in terms of the affinity constant (KA), which corresponds to 1/KD. The binding affinity of an anti- MCL-1 polypeptide can be determined in a manner known to the skilled person, depending on the specific target protein of interest. A KD value greater than about 1 millimolar is generally considered to indicate non-binding or non-specific binding.

An anti-MCL-1 polypeptide as envisaged herein is said to be 'specific for a first target protein of interest as opposed to a second target protein of interest' when it binds to the first target protein of interest with an affinity that is at least 5 times, such as at least 10 times, such as at least 100 times, and preferably at least 1000 times higher than the affinity with which that polypeptide binds to the second target protein of interest. Accordingly, in certain embodiments, when a polypeptide is said to be 'specific for' a first target protein of interest as opposed to a second target protein of interest, it may specifically bind to (as defined herein) the first target protein of interest, but not to the second target protein of interest.

The 'half-life' of an anti- MCL-1 polypeptide can generally be defined as the time that is needed for the *in vivo* serum or plasma concentration of the polypeptide to be reduced by 50%. The *in vivo* half-life of an anti- MCL-1 polypeptide can be determined in any manner known to the person skilled in the art, such as by pharmacokinetic analysis. As will be clear to the skilled person, the half-life can be expressed using parameters such as the t_{1/2}-alpha, t_{1/2}-beta and the area under the curve (AUC). An increased half-life *in vivo* is generally characterized by an increase in one or more and preferably in all three of the parameters t_{1/2}-alpha, t_{1/2}-beta and the area under the curve (AUC).

As used herein, the terms 'inhibiting', 'reducing' and/or 'preventing' may refer to (the use of) an anti-MCL-1 polypeptide that specifically binds to MCL-1 and inhibits, reduces and/or prevents the interaction between MCL-1 and a pro-apoptotic protein, preferably between MCL-1 and the pro-apoptotic protein Bc1-2 homologous antagonist killer (BAK).

The terms 'treat' or 'treatment' encompass both the therapeutic treatment of an already developed disease or condition, such as the therapy of an already developed MCL-1-mediated disease, as well as prophylactic or preventive measures, wherein the aim is to prevent or lessen the chances of incidence of an undesired affliction, such as to prevent occurrence, development and progression of a MCL-1-mediated disease. Beneficial or desired clinical results may include, without limitation, alleviation of one or more symptoms or one or more biological markers, diminishment of extent of disease, stabilized (i.e., not worsening) state of disease, delay or slowing of disease progression, amelioration or palliation of the disease state, and the like. 'Treatment' can also mean prolonging survival as compared to expected survival if not receiving treatment.

As used herein, the terms 'subject', 'individual' or 'patient' can be used interchangeably herein, and typically and preferably denote humans, but may also encompass reference to non-human animals, preferably warm-blooded animals, even more preferably mammals, such as, e.g., non-human primates, rodents, canines, felines, equines, ovines, porcines, and the like. The term 'non-human animals' includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. In certain embodiments, the subject is a non-human mammal. In certain embodiments, the subject is a human subject. The term does not denote a particular age or sex. Thus, adult and new-born subjects, as well as foetuses, whether male or female, are intended to be covered. Examples of subjects include humans, dogs, cats, cows, goats, and mice.

Suitable subjects may include without limitation subjects presenting to a physician for a screening for a MCL-1-mediated disease or disorder, subjects presenting to a physician with symptoms and signs indicative of a MCL-1-mediated disease or disorder, subjects diagnosed with a MCL-1-mediated disease or disorder, subjects who have received therapy for a MCL-1-mediated disease or disorder, subjects undergoing treatment for a MCL-1-mediated disease or disorder, and subjects having a MCL-1-mediated disease or disorder in remission. Methods for diagnosing a MCL-1-mediated disease or disorder are known in the art and include microscopic analysis of a sample (biopsy) of the affected area of the tissue or organ, such as a skin biopsy.

Present inventors designed new polypeptides directly binding to MCL-1, more particularly to the amino acid region human MCL-1 (hMCL-1) 163-327 (SEQ ID NO: 6) or the amino acid region hMCL-1 172-327 (SEQ ID NO: 40). The polypeptides as envisaged herein comprise at least one Alphabody structure with a MCL-1 binding region comprising a sequence LRXVGDXV (SEQ ID NO: 1), wherein X can be any amino acid, i.e. the key binding determinants inspired by the interaction of MCL-1with its inhibitory BH3 helix and other peptides, and further comprising an albumin binding region comprising a sequence SDFYFXXINKA (SEQ ID NO: 2) and a sequence TXEXVXALKXXILXAH (SEQ ID NO: 3), wherein X can be any amino acid. The polypeptides as envisaged herein have the ability to bind MCL-1 with affinities lying in the picomolar range, are capable of neutralizing the biological activity of MCL-1 and have a significantly extended serum half-life. In addition, present inventors also designed and successful implemented a cellular uptake sequence motif comprising a sequence (RP)ₙ, wherein n is an integer from 6 to 9, that enables efficient translocation of a polypeptide comprising at least one Alphabody structure across the cell membrane to reach an intracellular target without loss of binding specificity, such as enabling the MCL-1 binding polypeptide across the cell membrane to reach intracellular MCL-1. Moreover, the polypeptides as envisaged herein can be used in the treatment of multiple cancer types by inducing cell growth inhibition, apoptosis and/or cell cycle arrest in cancer cells. More particularly, present inventors have shown that the polypeptides as envisaged herein alleviate tumour burden in xenograft mice tumour models using MM cells, as well as primary T-ALL cells from patients.

Moreover, the MCL-1-binding polypeptides as envisaged herein, which comprise an amino acid sequence of at least one Alphabody, maintain the advantages identified for Alphabody scaffolds. Alphabodies not only have a unique structure but also have several advantages over the traditional (immunoglobulin and non-immunoglobulin) scaffolds known in the art. These advantages include, but are not limited to, the fact that they are compact and small in size (between 10 and 14 kDa, which is about 10 times smaller than an antibody), they are extremely thermostable (i.e., they generally have a melting temperature of more than 100 °C), they can be made resistant to different proteases, they are highly engineerable (in the sense that multiple substitutions will generally not obliterate their correct and stable folding), and have a structure which is based on natural motifs which have been redesigned via protein engineering techniques. Furthermore, Alphabody polypeptides typically have a triple-stranded alpha-helical coiled coil structure which is suitable as a scaffold structure. An Alphabody polypeptide surface consists of pre-shaped regions such as concave grooves, convex alpha-helical surfaces, and flexible linker regions. Any such region, or a combination thereof, can be modified for different purposes, including the design or selection of target binding sites, modification of global charge and/or polarity and/or posttranslational modifications. In addition, the termini of the Alphabody polypeptide can be appended with different tags, such as recognition tags or cell-penetrating peptide sequence motifs for intracellular delivery.

The polypeptides as envisaged herein comprise an amino acid sequence, which corresponds to the structural motif of an Alphabody. Generally, Alphabodies as envisaged herein have an amino acid sequence having the general formula
HRS1-L1-HRS2-L2-HRS3,
- wherein each of HRS1, HRS2 and HRS3 is independently a heptad repeat sequence (HRS) comprising or consisting of 2 to 7 consecutive heptad repeat units,
- wherein said heptad repeat units are 7-residue (poly)peptide fragments represented as 'abcdefg' or 'defgabc', wherein the symbols 'a' to 'g' denote conventional heptad positions,
- wherein at least 50% of all heptad a- and d-positions are occupied by isoleucine residues,
- wherein each HRS starts and ends with an aliphatic or aromatic amino acid residue located at a heptad a- or d-position,
- wherein each of L1 and L2 are independently a linker fragment, which covalently connect HRS1 to HRS2 and HRS2 to HRS3, respectively.

HRS 1 is referred to herein as alpha-helix A or the first alpha-helix of the polypeptide as envisaged herein, HRS2 is referred to herein as alpha-helix B or the second alpha-helix of the polypeptide as envisaged herein, and HRS3 is referred to herein as alpha-helix C or the third alpha-helix of the polypeptide as envisaged herein.

The Alphabodies as envisaged herein may be adapted to increase the solubility thereof. For example, an N-terminal polar terminal core layer (nPTC), which shields the hydrophobic isoleucine residues located in the core of the Alphabody from the solvent, could be introduced into the polypeptides as envisaged herein. The engineered polar core layer serves a two-fold purpose to facilitate interaction of the core of the polypeptide as envisaged herein with MCL-1, and to mediate compliance with the conformational criteria imposed by the binding target.

In more particular embodiments, the heptad a position of the first heptad repeat of HRS1 is occupied by threonine (T), the heptad d position of the fourth heptad repeat of HRS2 is occupied by glutamine (Q), and the heptad a position of the first heptad repeat of HRS3 is occupied by threonine (T). The threonine at the heptad a-position of the first heptad repeat unit of HRS 1 and HRS3 can form together with the glutamine at the heptad d-position of the fourth heptad repeat unit of HRS2 a polar terminal core layer at the N-terminus, herein also referred to as 'nPTC'.

It has been found that Alphabodies comprising a specific sequence motif LRXVGDXV (SEQ ID NO: 1), wherein X can be any amino acid, preferably within HRS2, are capable of neutralizing the activity of MCL-1. The presence of this sequence motif mediates the binding to MCL-1, which results in a neutralizing effect. The binding of the polypeptides as envisaged herein to MCL-1 can be attributed to the presence of six specific amino acids at specific positions relative to each other in the HRS2 of the Alphabody. Indeed, it has surprisingly been found that a relatively limited number of amino acid positions contribute significantly to the binding to MCL-1. The polypeptides as envisaged herein achieve their specific binding to MCL-1 on the one hand by electrostatic interactions mediated by the arginine (R) and aspartic acid (D) residues within the binding motif and on the other hand by hydrophobic interactions mediated by the leucine (L) and two valine (V) residues within this motif.

The person skilled in the art will understand that this sequence motif is preferably incorporated into one of the helices of the Alphabody, such that no other part of the Alphabody is in sterical conflict with MCL-1. Suitable locations for incorporation of the sequence motif into the Alphabody can be determined using structural modelling analysis.

More particularly, it has been found that polypeptides comprising at least one Alphabody structure comprising a sequence LRXVGDXV (SEQ ID NO: 1), wherein X can be any amino acid, can provide neutralizing activity towards MCL-1. Accordingly, in particular embodiments, the polypeptide as taught herein comprises an Alphabody structure, wherein HRS2 comprises a MCL-1 binding region comprising a sequence LRXVGDXV (SEQ ID NO: 1), wherein X can be any amino acid.

Preferably, the sequence LRXVGDXV (SEQ ID NO: 1), wherein X can be any amino acid, is positioned within the Alphabody structure, and particularly within HRS2, to allow to form an amphipathic helical element with hydrophobic residues Leuₙ, Valₙ₊₃ and Valₙ₊₇ on one face and Argₙ₊₁ and Aspₙ₊₅ projecting a polar helical face on the Alphabody structure. The term "n+x", wherein x is any number from 1 to 7, indicates the position of the amino acid vis-a-vis the first amino acid (i.e. Leuₙ) of the MCL-1 binding sequence motif.

In particular embodiments, HRS2 comprises a MCL-1 binding region comprising, consisting essentially of, or consisting of a sequence LRIVGDQV (SEQ ID NO: 7).

In particular embodiments, in HRS2:
(i) the heptad f-position of the second heptad repeat unit is occupied by leucine;
(ii) the heptad g-position of the second heptad repeat unit is occupied by arginine;
(iii) the heptad b-position of the third heptad repeat unit is occupied by valine;
(iv) the heptad c-position of the third heptad repeat unit is occupied by glycine;
(v) the heptad d-position of the third heptad repeat unit is occupied by aspartate; and/or (preferably and)
(vi) the heptad f-position of the third heptad repeat unit is occupied by valine.

In particular embodiments, if the aspartic acid (D) of the LRXVGDXV (SEQ ID NO: 1) motif is located at the heptad d-position of the third heptad repeat unit of HRS2, then the heptad a-position of the second heptad repeat unit of HRS1 is preferably occupied by threonine. This may allow these residues to cluster together in the same core layer. Clustering of threonine and aspartate into the same core layer provides a more stable conformation of the Alphabody structure.

In particular embodiments, HRS2 comprises, consists essentially of, or consists of a sequence with at least 70%, at least 75%, at least 80%, at least 85%, or at least 90%, preferably at least 95% (such as 95%, 96%, 97%, 98% or 99%), more preferably at least 99%, or even more preferably 100% sequence identity to a sequence IEEITKQIAAIQLRIVGDQVQIAYQT (SEQ ID NO: 8).

In addition to its binding to MCL-1, the polypeptides as envisaged herein bind to the serum protein albumin. More particularly, the polypeptides as envisaged herein have been chemically modified to increase the biological or plasma half-life thereof, by means of the addition of a group that binds to serum albumin.

In particular embodiments, the polypeptides as envisaged herein bind to human serum albumin. By means of an example, human albumin preproprotein sequence is annotated under NCBI Genbank (http://www.ncbi.nlm.nih.gov/) accession number NP_000468.1 and Uniprot accession number P02768.2 and the mature human albumin protein sequence is annotated under Uniprot accession number P02768.

In particular embodiments, the polypeptide as taught herein comprises an albumin binding region conjugated to the C-terminus of HRS3 of the Alphabody structure, wherein said albumin binding region comprises a sequence SDFYFXXINKA (SEQ ID NO: 2; also referred to herein as the fourth alpha-helix of the polypeptide as envisaged herein or as alpha-helix D) and a sequence TXEXVXALKXXILXAH (SEQ ID NO: 3; also referred to herein as the fifth alpha-helix of the polypeptide as envisaged herein or as alpha-helix E), wherein X can be any amino acid. Present inventors found that the conjugation of such albumin binding region to the C-terminus of HRS3 allows achieving a half-life of the polypeptide as envisaged herein that is about 10-fold higher than a similar polypeptide without the albumin binding region. Furthermore, as the fourth and fifth alpha-helices are small, an albumin-binding polypeptide can be obtained without significantly increasing the molecular weight of the polypeptide. Furthermore, the albumin binding region does not affect the thermal stability and target affinity of the polypeptide as envisaged herein.

In particular embodiments, the sequence as defined by SEQ ID NO: 3 is located downstream (i.e. C-terminally) of the sequence as defined by SEQ ID NO: 2.

The albumin binding region in the polypeptide as envisaged herein can be produced by grafting the alpha-helices of *Finegoldia magna* (*F. magna*) protein G, for example as described in Johnasson et al., Structure, specificity, and mode of interaction for bacterial albumin-binding, Journal of Biological Chemistry, 2002, 277, 8114-8120, onto the Alphabody structure, as described elsewhere herein. In particular embodiments, the protein G comprises, consists essentially of or consists of a sequence TIDQWLLKNAKEDAIAELKKAGITSDFYFNAINKAKTVEEVNALKNEILKAHA (SEQ ID NO: 9), wherein the sequence AKEDAIAELK (SEQ ID NO: 10) forms a first alpha-helix, the sequence DFYFNAIN (SEQ ID NO: 11) forms a second alpha-helix and the sequence EEVNALKNEILKA (SEQ ID NO: 12) forms a third alpha-helix.

In addition, the sequences as defined by SEQ ID NO: 2 and SEQ ID NO: 3 are each, independently, able to form an alpha-helix and can form together with the Alphabody structure a five-stranded alpha-helical bundle. Accordingly, in particular embodiments, HRS1, HRS2, HRS3 and the albumin binding region together form a five-stranded alpha-helical bundle.

The present inventors found that the variable amino acids in the sequences defined by SEQ ID NO: 2 and SEQ ID NO: 3 can be specifically engineered to optimize packing between HRS3 and the α-helices of the albumin binding region, to improve polarity, and/or to modulate affinity of the polypeptide for different albumins, such as based on modelling expertise.

For example, the introduction of an alanine (A) at amino acid position 4 of the amino acid sequence defined by SEQ ID NO: 3 can reduce the affinity of the polypeptide as taught herein for serum albumin and improve its ability to cross the lipid bilayer of a cell. Accordingly, in particular embodiments, said albumin binding region comprises, consists essentially of, or consists of a sequence SDFYFXXINKA (SEQ ID NO: 2) and a sequence TXEAVXALKXXILXAH (SEQ ID NO: 13), wherein X can be any amino acid.

In particular embodiments, the albumin binding region is covalently linked to HRS3 (of the Alphabody structure) by a disulphide bond. Tethering of the albumin binding region to HRS3 allows enhancing the stability of the polypeptide.

In particular embodiments, the albumin binding region comprises, consists essentially of, or consists of a sequence SDFYFXXINKA (SEQ ID NO: 2) and a sequence TCEAVXALKXXILXAH (SEQ ID NO: 14) and the HRS3 (of the Alphabody structure) comprises a cysteine at the heptad b-position of the third heptad repeat unit. In particular embodiments, the cysteine at position 2 of the amino acid sequence as defined by SEQ ID NO: 14 and the cysteine at the heptad b-position of the third heptad repeat unit of HRS3 form together a disulphide bond.

The sequence as defined by SEQ ID NO: 2 and the sequence as defined by SEQ ID NO: 3, wherein X can be any amino acid, can be linked to each other by any method known in the art to connect two amino acid sequences, such as by an amino acid residue, preferably by a lysine (K) residue. Accordingly, in particular embodiments, the albumin binding region comprises, consists essentially of, or consists of a sequence SDFYFXXINKAKTXEXVXALKXXILXAH (SEQ ID NO: 4), a sequence SDFYFXXINKAKTXEAVXALKXXILXAH (SEQ ID NO: 15), a sequence SDFYFXXINKAKTCEXVXALKXXILXAH (SEQ ID NO: 16), or a sequence SDFYFXXINKAKTCEAVXALKXXILXAH (SEQ ID NO: 5), wherein X can be any amino acid.

In particular embodiments, the albumin binding region comprises, consists essentially of, or consists of a sequence with at least 70%, at least 75%, at least 80%, at least 85%, or at least 90%, preferably at least 95% (such as 95%, 96%, 97%, 98% or 99%), more preferably at least 99%, or even more preferably 100% sequence identity to a sequence SDFYFELINKAKTCEAVEALKEHILAAH (SEQ ID NO: 17).

In particular embodiments, HRS3 and the albumin binding region are linked to each other by a linker region, preferably by a linker region comprising, essentially consisting of or consisting of a sequence ELKXAGIT (SEQ ID NO: 18), wherein X can be any amino acid, more preferably by a linker region comprising, essentially consisting of or consisting of a sequence ELKEAGIT (SEQ ID NO: 19). The linker region as defined by SEQ ID NO: 18 or 19 will form a loop between the alpha-helix HRS3 and the alpha-helix with a sequence as defined by SEQ ID NO: 2 or 3, preferably by SEQ ID NO: 2.

In particular embodiments, HRS3 and the sequence as defined by SEQ ID NO: 2 or 3, preferably by SEQ ID NO: 2, are linked to each other by a linker region, preferably by a linker region comprising, essentially consisting of or consisting of a sequence ELKXAGIT (SEQ ID NO: 18), wherein X can be any amino acid, more preferably by a linker region comprising, essentially consisting of or consisting of a sequence ELKEAGIT (SEQ ID NO: 19).

It has been determined that the remaining positions of the Alphabody structure of the polypeptides are less critical. More particularly, it has been found that in the polypeptides having improved properties as envisaged herein, the sequence of the HRS1 or HRS3 heptad repeat is of limited importance. Exemplary sequences of Alphabody structures, more particularly, of the backbone of the Alphabody template, are those as described in Desmet J. et al., Structural basis of IL-23 antagonism by an Alphabody protein scaffold, Nature Communications, 2014, 5, Article number: 5237, or the like, such as a backbone template essentially consisting of heptad repeats of the amino acid sequence IAAIQKQ (SEQ ID NO: 20).

Accordingly, in particular embodiments, HRS1 comprises, consists essentially of, or consists of a sequence with at least 70%, at least 75%, at least 80%, at least 85%, at least 90%, at least 95% (such as 95%, 96%, 97%, 98% or 99%), at least 99%, or 100% sequence identity to a sequence TEQIQKRTAAIQKRIAAIQKRIYAMT (SEQ ID NO: 36).

In particular embodiments, HRS3 comprises a cysteine at heptad b-position of the third heptad repeat unit. This cysteine can form a disulphide bond with the fifth alpha-helix of the polypeptide as envisaged herein, when it comprises a sequence as defined in SEQ ID NO: 14.

In particular embodiments, HRS3 comprises, consists essentially of, or consists of a sequence with at least 70%, at least 75%, at least 80%, at least 85%, at least 90%, at least 95% (such as 95%, 96%, 97%, 98% or 99%), at least 99%, or 100% sequence identity to a sequence TEEIQKQIAAIETQICKIEAAI (SEQ ID NO: 21), preferably HRS3 consists of a sequence as defined by SEQ ID NO: 21.

The polypeptides envisaged herein contain linkers connecting the subsequent heptad repeat sequences HRS1, HRS2, HRS3. It is envisaged that the nature of the linker is not critical with regard to the binding properties of the polypeptides envisaged herein. In particular embodiments, the linker sequences are glycine and serine-rich sequences with a minimum length of about 8 amino acids. In particular embodiments the linkers comprise 1 or 2 repeats of a "glycine/serine-rich" sequence such as GGSGGSGG (SEQ ID NO: 22) or GSGGGGSG (SEQ ID NO: 23). The linker connecting HRS1 and HRS2, referred to as "linker 1", may be the same or different from the linker connecting HRS2 and HRS3, referred to as "linker 2". In particular embodiments, linker 1 is (GGSGGSGG)n, wherein n = 1 or 2 and linker2 is (GSGGGGSG)n, wherein n = 1 or 2. Such linkers need not be composed only of Gly/Ser residues and in addition typically contain one or more amino acids N- and/or C-terminally thereof. Such linkers include, but are not limited to TGGSGGSGGMS (SEQ ID NO: 24) and TGGSGGSGGGGSGGSGGMS (SEQ ID NO: 25).

The polypeptides as envisaged herein may comprise at least one sequence comprising an Alphabody structure, a MCL-1 binding region and an albumin binding region; and optionally one or more further groups, moieties, residues optionally linked via one or more linkers.
In particular embodiments, the polypeptides as envisaged herein thus may optionally contain one or more further groups, moieties or residues for binding to other targets or target proteins of interest. It should be clear that such further groups, residues, moieties and/or binding sites may or may not provide further functionality to the Alphabodies as envisaged herein (and/or to the polypeptide or composition in which it is present). Such groups, residues, moieties or binding units may also for example be chemical groups which can be biologically and/or pharmacologically active.

These groups, moieties or residues are, in particular embodiments, linked N-terminally to the Alphabody or C-terminally to the albumin binding region. In particular embodiments however, one or more groups, moieties or residues are linked to the body of the Alphabody, e.g. to a free cysteine in an alpha-helix or in a linker region.

The polypeptides envisaged herein may further comprise additional amino acids or moieties covalently bound to the sequence corresponding to HRS1-L1-HRS2-L2-HRS3, such as N-terminally attached to the first heptad repeat sequence, HRS1, or C-terminally attached to the last heptad repeat sequence, HRS3, or C-terminally attached to the albumin binding region formed by alpha-helices 4 and 5.. In particular embodiments, these additional amino acids ensure a particular function other than MCL-1 binding. In particular embodiments, the number of amino acids added will be between 1-15 amino acids. Examples of additional amino acids and their functions are described herein below.

Additionally or alternatively, the optional N-terminal and C-terminal extensions can be, for example, a tag for detection or purification (e.g., a His-tag) or another protein or protein domain, in which case the full construct is denoted a fusion protein. For the sake of clarity, the optional extensions N and C are herein considered not to form part of a single-chain Alphabody structure, which is defined by the general formula 'HRS1-L1-HRS2-L2-HRS3'.

At least one Alphabody polypeptide can be fused with a cell penetrating peptide N- and/or C-terminally to deliver the resulting polypeptides effectively into the cell and remain stable within the cell while allowing high-affinity binding to a target inside the cell.

Accordingly, in particular embodiments, polypeptides are provided comprising or essentially consisting of at least one Alphabody directed against MCL-1, fused with or conjugated to a group, moiety or peptide, thereby ensuring efficient cell penetration of the polypeptide, whereby stability of the polypeptide is maintained within the cell allowing efficient binding to an intracellular target.

In certain particular embodiments, the polypeptides provided herein, comprise at least one Alphabody directed against MCL-1 which is linked, i.e. coupled, fused or conjugated, either directly or indirectly with a group, (protein) moiety or peptide so as to allow or ensure cell penetration of the polypeptide.

In certain specific embodiments, the polypeptides as envisaged herein comprise at least one Alphabody structure that can bind to MCL-1, which is linked directly to a group, (protein) moiety or peptide allowing cell penetration.

In alternative particular embodiments, the group, (protein) moiety or peptide, which allows the polypeptides as envisaged herein to enter cells can also be linked to an entity, other than the Alphabody directed against MCL-1, as long as this entity forms part of the polypeptide. Such other entity (to which the group, moiety or peptide that allows cell penetration can be linked) can for example be a second Alphabody (e.g. in the case of bivalent or multivalent polypeptides comprising concatenated Alphabodies) or can for example be a group or protein, which has a specific function, such as for example a protein extending the in vivo half-life of the polypeptide, or a targeting peptide, which targets or directs the polypeptide to certain specific cell types. Also, the other entity can be a linker, such as a suitable peptidic linker to couple proteins, as known by the person skilled in the art.

In further particular embodiments, polypeptides are provided comprising or essentially consisting of at least one Alphabody directed against MCL-1, which Alphabody is conjugated to a cell penetrating peptide (CPP), such as but not limited to TAT, CPP5, Penetratin, Pen-Arg, pVEC, M918, TP10, (Madani et al, Journal of Biophysics, Volume 2011, Article ID 414729), TAT-HA fusogenic peptides (Wadia et al., Nat Med, 2004, 10, 310-315).

Present inventors have designed and successfully implemented a cellular uptake sequence motif consisting of a series of arginine and proline residues that enables efficient translocation of the polypeptide comprising at least one Alphabody, such as the polypeptide as envisaged herein, without loss of binding specificity to the target, such as MCL-1.

Accordingly, provided herein is a polypeptide capable of specifically binding to a target comprising at least one Alphabody, wherein said Alphabody is conjugated to at least one, such as one or two, peptide(s) for facilitating cellular entry of the polypeptide, wherein said peptide(s) comprise(s), consist(s) essentially of or consist(s) of, a sequence (RP)ₙ, wherein n is an integer from 6 to 9, such as 6, 7, 8 or 9, preferably 7, 8 or 9, more preferably 7.

The inventors have moreover found that the provision of an albumin binding region and a peptide for facilitating cellular entry (e.g. cellular uptake sequence motif) as described herein provides optimal stability without compromising the ability to enter into the cell.

Accordingly in particular embodiments, a polypeptide comprising an Alphabody against an intracellular target encompasses both the albumin binding region as described elsewhere in the present specification and the peptide for facilitating cellular entry (e.g. cellular uptake sequence motif) as described elsewhere in the present specification. In particular embodiments, polypeptides are provided comprising or essentially consisting of at least one Alphabody directed against MCL-1, as taught herein, wherein said Alphabody is conjugated to at least one, such as one or two, peptide(s) for facilitating cellular entry of the polypeptide, wherein said peptide(s) comprise(s), consist(s) essentially of or consist(s) of a sequence (RP)ₙ, wherein n is an integer from 6 to 9, such as 6, 7, 8 or 9, preferably 7, 8 or 9, more preferably 7.

In particular embodiments, the polypeptide or Alphabody is conjugated to at least one, such as one or two, (RP)₇ cationization motif(s) (i.e. a peptide consisting of an amino acid sequence RPRPRPRPRPRPRP (SEQ ID NO: 26)). In particular embodiments, polypeptides are provided comprising at least one Alphabody specifically binding to MCL-1, which polypeptides are characterized by the presence of a sequence conjugated to the Alphabody structure sequence ensuring internalization of the polypeptide into the cell. In particular embodiments, the polypeptides envisaged herein are characterized by the presence of a sequence such as, but not limited to, RPRPRPRPRPRPRP (SEQ ID NO: 26). In particular embodiments, the sequence is a (RP)₇ cationization motif.

In particular embodiments, said peptide for facilitating cellular entry of the polypeptide is conjugated to the N- and/or C-terminus of the polypeptide. In more particular embodiments, said peptide for facilitating cellular entry of the polypeptide is conjugated to the N-terminus of HRS1 and/or to the C-terminus of the albumin binding region. In particular embodiments, a first peptide for facilitating cellular entry of the polypeptide is conjugated to N-terminus of HRS1 and a second peptide for facilitating cellular entry of the polypeptide is conjugated to the C-terminus of HRS3. In more particular embodiments, a first peptide for facilitating cellular entry of the polypeptide is conjugated to N-terminus of HRS1 and a second peptide for facilitating cellular entry of the polypeptide is conjugated to the C-terminus of the albumin binding region.

In particular embodiments, the polypeptides of the present invention comprise amino acids that have been chemically modified. For example, such a modification may involve the introduction or linkage of one or more functional groups, residues or moieties into or onto the amino acid sequence. These groups, residues or moieties may confer one or more desired properties or functionalities to the polypeptides. Examples of such functional groups will be clear to the skilled person as detailed below.
For example, the introduction or linkage of such functional groups in a polypeptide as envisaged herein can result in an increase in the solubility and/or the stability of the polypeptide or in a reduction of the toxicity of the polypeptide, or in the elimination or attenuation of any undesirable side effects of the polypeptide, and/or in other advantageous properties.
A particular modification of the polypeptides envisaged herein may comprise the introduction of one or more detectable labels or other signal-generating groups or moieties, depending on the intended use of the labelled polypeptide.

Yet a further particular modification may involve the introduction of a chelating group, for example to chelate one or more metals or metallic cations.
A particular modification may comprise the introduction of a functional group that is one part of a specific binding pair, such as the biotin-(strept)avidin binding pair.
Other potential chemical and enzymatic modifications will be clear to the skilled person.

In particular embodiments, the polypeptides as envisaged herein comprise an amino acid sequence that is capable of specifically binding to MCL-1, more preferably hMCL-1 with an amino acid sequence as set forth in SEQ ID NO: 37. By means of an example, human MCL-1 amino acid sequence is annotated under NCBI Genbank (http://www.ncbi.nlm.nih.gov/) accession number NP_068779.1 (isoform 1), NP_877495.1 (isoform 2) or NP_001184249.1 (isoform 3), preferably NP_068779.1 (isoform 1), and Uniprot accession number Q07820.3.

In particular embodiments, the Alphabodies envisaged herein are specific for the BH3-binding groove of MCL-1, more particularly the Alphabodies envisaged herein are specific for the amino acid region hMCL-1 163-327 (SEQ ID NO: 6) or the amino acid region hMCL-1 172-327 (SEQ ID NO: 40) of the human MCL-1 amino acid sequence annotated under NCBI Genbank (http://www.ncbi.nlm.nih.gov/) accession number NP_068779.1 (isoform 1). Binding of the Alphabodies envisaged herein to the BH3-binding groove of MCL-1 is necessary and sufficient to prevent binding of pro-apoptotic proteins, such as BAK, to MCL-1.

In particular embodiments, the polypeptides as envisaged herein will bind to MCL-1 with a dissociation constant (KD) of less than about 10 picomolar (10 pM). A KD value greater than about 1 millimolar is generally considered to indicate non-binding or non-specific binding.

MCL-1's anti-apoptotic function is achieved by sequestering the pro-apoptotic protein BAK through the BH3 domain containing hydrophobic groove. In the anti-apoptotic (i.e. pro-survival) state, BAK interacts with MCL-1 and is unable to execute the apoptotic program, thereby allowing cells to maintain homeostasis. In the apoptotic state, such as upon stress, BAK are released from MCL-1 as a result of BH3-only proteins (e.g. BIM, PUMA, NOXA) binding to MCL-1. After dissociation from pro-survival proteins, BAK exposes its BH3 domain through which it forms oligomers that are able to induce release of cytochrome c and the activation of initiator caspases. MCL-1 is frequently overexpressed in human cancers leading to the immortalization of said cancers. Therefore, inhibiting the interaction between MCL-1 and BAK can result in a cell killing effect and can elicit a therapeutically relevant outcome.

The polypeptides envisaged herein that specifically bind to MCL-1 are moreover capable of specifically inhibiting, preventing or decreasing the activity of MCL-1, and/or of inhibiting, preventing or decreasing the signalling and biological mechanisms and pathways in which MCL-1 plays a role. Indeed by binding to MCL-1, the polypeptides envisaged herein can prevent or inhibit the interaction between MCL-1 and pro-apoptotic proteins, such as BAK. Accordingly, the polypeptides and pharmaceutical compositions comprising said polypeptides envisaged herein can be used to affect, change or modulate MCL-1-mediated diseases and disorders, such as those responsive to the induction of apoptotic cell death (e.g. disorders characterized by dysregulation of apoptosis) including different types of cancer (such as multiple myeloma).

More particularly, 'inhibiting', 'reducing' and/or 'preventing' using the polypeptides envisaged herein may mean either inhibiting, reducing and/or preventing the interaction between MCL-1 and BAK, and/or preventing one or more biological or physiological mechanisms, effects, responses, functions pathways or activities in which MCL-1 is involved, such as by at least 10%, but preferably at least 20%, for example by at least 50%, at least 60%, at least 70%, at least 80%, at least 90%, at least 95% or more, as measured using a suitable *in vitro,* cellular or *in vivo* assay, compared to the activity of MCL-1 in the same assay under the same conditions but without using said polypeptide.

The capability of the polypeptides envisaged herein of inhibiting, preventing or decreasing the activity of MCL-1 may be reflected in the capability of the polypeptides envisaged herein of inhibiting, preventing or decreasing binding between MCL-1 and BAK. The binding between MCL-1 and BAK may be determined by all methods known in the art to determine protein:protein interactions, such as co-immunoprecipitation and western blot analysis. Furthermore, as a decreased binding between MCL-1 and pro-apoptotic proteins BAK will lead to an increased activity of BAK, an inhibited, prevented or decreased binding between MCL-1 and BAK may also be determined by co-immunoprecipitation assays, cell death assays, apoptosis assays, such as cytochrome c release assays or flow cytometry assays (e.g. for detecting changes in the plasma and mitochondrial membranes, caspase activity, nuclear condensation, and fragmentation), or may be determined by BAK activation assays using antibodies specifically recognizing activated BAK.

The application also provides pharmaceutical compositions comprising one or more polypeptides and/or nucleic acid sequences as envisaged herein and optionally at least one pharmaceutically acceptable carrier (also referred to herein as pharmaceutical compositions of the invention). According to certain particular embodiments, the pharmaceutical compositions of the invention may further optionally comprise at least one other pharmaceutically active compound.

The pharmaceutical can be used in the prevention and/or treatment of MCL-1-mediated diseases and disorders, such as those responsive to the induction of apoptotic cell death (e.g. disorders characterized by dysregulation of apoptosis) including different types of cancer (such as multiple myeloma).

In particular, the present invention provides pharmaceutical compositions comprising polypeptides as envisaged herein that are suitable for prophylactic and/or therapeutic use in a warm-blooded animal, and in particular in a mammal. In particular embodiments, the mammal is a human, but veterinary purposes are also envisaged.
Generally, for pharmaceutical use, the polypeptides envisaged herein may be formulated as a pharmaceutical preparation or compositions comprising at least one polypeptide as envisaged herein and at least one pharmaceutically acceptable carrier, diluent or excipient and/or adjuvant, and optionally one or more further pharmaceutically active polypeptides and/or compounds. Such a formulation may be suitable for oral, parenteral, topical administration or for administration by inhalation. Thus, the compositions envisaged herein can for example be administered orally, intraperitoneally, intravenously, subcutaneously, intramuscularly, transdermally, topically, by means of a suppository, by inhalation, again depending on the specific pharmaceutical formulation or composition to be used.
The pharmaceutical compositions may also contain suitable binders, disintegrating agents, sweetening agents or flavouring agents. Tablets, pills, or capsules may be coated for instance with gelatin, wax or sugar and the like. In addition, the compositions may be formulated and/or administered using sustained-release formulations and devices.
The compositions envisaged herein will be administered in an amount which will be determined by the medical practitioner based inter alia on the severity of the condition and the patient to be treated. Typically, for each disease indication an optical dosage will be determined specifying the amount to be administered per kg body weight per day, either continuously (e.g. by infusion), as a single daily dose or as multiple divided doses during the day. The clinician will generally be able to determine a suitable daily dose, depending on the factors mentioned herein. It will also be clear that in specific cases, the clinician may choose to deviate from these amounts, for example on the basis of the factors cited above and his/her expert judgment.
In particular, the polypeptides envisaged herein may be used in combination with other pharmaceutically active compounds or principles that are or can be used for the prevention and/or treatment of the diseases and disorders cited herein, as a result of which a synergistic effect may or may not be obtained. Examples of such compounds and principles, as well as routes, methods and pharmaceutical formulations or compositions for administering them will be clear to the clinician.

Also envisaged herein is the use of the polypeptides envisaged herein for use as a medicament. Also envisaged herein is the use of the polypeptides envisaged herein for the preparation of a medicament for the prevention and/or treatment of at least one MCL-1-mediated disease and/or disorder in a subject. Accordingly, the invention provides the polypeptides envisaged herein, or the pharmaceutical compositions comprising the polypeptides envisaged herein for use in the prevention and/or treatment of at least one MCL-1-mediated disease and/or disorder in a subject. In other words, provided herein are methods for the prevention and/or treatment of a MCL-1-mediated disease and/or disorder comprising administering to a subject in need thereof, a pharmaceutically active amount of one or more polypeptides and/or pharmaceutical compositions as envisaged herein.
The subject or patient to be treated is in particular a mammal, and more in particular a human suffering from, or at risk of and MCL-1-mediated disease or disorder. Examples of MCL-1-mediated diseases or disorders, such as those responsive to the induction of apoptotic cell death (e.g. disorders characterized by dysregulation of apoptosis), include a neoplastic disease, a hyperproliferative disorder, an inflammatory disease or disorder, an infectious disease or disorder, a cell cycle regulation disease or disorder, or an autophagy regulation disease or disorder, preferably a neoplastic disease or an autoimmune disease.Present inventors have shown that the polypeptides as envisaged herein are potent inhibitors of several MCL-1dependent oncogenic cell lines and are able to induce apoptosis in a dose-dependent manner by activating the BAK-dependent mitochondrial apoptotic pathway. Furthermore, treatment with the polypeptides as envisaged herein results in an induction of cell death via apoptosis in haematological malignancies such as MM, leukaemia and lymphoma.
Accordingly, the invention provides the polypeptides envisaged herein, or the pharmaceutical compositions comprising the polypeptides envisaged herein for use in the prevention and/or treatment of a neoplastic disease.

The term 'neoplastic disease' generally refers to any disease or disorder characterized by neoplastic cell growth and proliferation, whether benign (not invading surrounding normal tissues, not forming metastases), pre-malignant (pre-cancerous), or malignant (invading adjacent tissues and capable of producing metastases). The term neoplastic disease generally includes all transformed cells and tissues and all cancerous cells and tissues. Neoplastic diseases or disorders include, but are not limited to abnormal cell growth, benign tumours, premalignant or precancerous lesions, malignant tumours, and cancer. Examples of neoplastic diseases or disorders are benign, pre-malignant, or malignant neoplasms located in any tissue or organ, such as in the prostate, colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, or urogenital tract.

In certain embodiments of the methods or uses as taught herein, the neoplastic disease may be a tumour or may be characterized by the presence of a tumour.

As used herein, the terms 'tumour', 'solid tumour' or 'tumour tissue' refer to an abnormal mass of tissue that results from excessive cell division. A tumour or tumour tissue comprises tumour cells which are neoplastic cells with abnormal growth properties and no useful bodily function. Tumours, tumour tissue and tumour cells may be benign, pre-malignant or malignant, or may represent a lesion without any cancerous potential. A tumour or tumour tissue may also comprise tumour-associated non-tumour cells, e.g., vascular cells which form blood vessels to supply the tumour or tumour tissue. Non-tumour cells may be induced to replicate and develop by tumour cells, for example, the induction of angiogenesis in a tumour or tumour tissue.

In certain embodiments of the methods or uses as taught herein, the neoplastic disease may be cancer.

As used herein, the term 'cancer' refers to a malignant neoplasm characterized by deregulated or unregulated cell growth. The term 'cancer' includes primary malignant cells or tumours (e.g., those whose cells have not migrated to sites in the subject's body other than the site of the original malignancy or tumour) and secondary malignant cells or tumours (e.g., those arising from metastasis, the migration of malignant cells or tumour cells to secondary sites that are different from the site of the original tumour). The term 'metastatic' or 'metastasis' generally refers to the spread of a cancer from one organ or tissue to another non-adjacent organ or tissue. The occurrence of the neoplastic disease in the other non-adjacent organ or tissue is referred to as metastasis.

Examples of cancer include but are not limited to carcinoma, sarcoma, lymphoma, blastoma, and leukaemia or lymphoid malignancies. More particular examples of such cancers include without limitation: squamous cell cancer (e.g., epithelial squamous cell cancer), lung cancer including small-cell lung cancer, non-small cell lung cancer, adenocarcinoma of the lung, squamous carcinoma of the lung and large cell carcinoma of the lung, cancer of the peritoneum, hepatocellular cancer, gastric or stomach cancer including gastrointestinal cancer, pancreatic cancer, glioma, glioblastoma, cervical cancer, ovarian cancer, liver cancer, bladder cancer, hepatoma, breast cancer, colon cancer, rectal cancer, colorectal cancer, endometrial cancer or uterine carcinoma, salivary gland carcinoma, kidney or renal cancer, prostate cancer, vulvar cancer, thyroid cancer, hepatic carcinoma, anal carcinoma, penile carcinoma, as well as CNS cancer, melanoma, head and neck cancer, bone cancer, bone marrow cancer, duodenum cancer, oesophageal cancer, thyroid cancer, or haematological cancer.

In certain embodiments of the methods or uses as taught herein, the neoplastic disease may be carcinoma, sarcoma or a solid tumour.

The term 'carcinoma' refers to a category of types of cancer that develop from epithelial cells. For example, a carcinoma is a cancer that begins in a tissue that lines the inner or outer surfaces of the body, and that arises from cells originating in the endodermal, mesodermal or ectodermal germ layer during embryogenesis.

The term 'sarcoma' refers to a category of types of cancer that arise from transformed cells of mesenchymal (connective tissue) origin. Connective tissue includes bone, cartilage, fat, vascular, or hematopoietic tissues, and sarcomas can arise in any of these types of tissues.

In particular embodiments, the neoplastic disease is a neoplastic disease characterized by increased expression of MCL-1, such as expression of MCL-1 which is increased in the neoplastic cell or tissue compared to a control (e.g. healthy) cell or tissue. Expression of MCL-1 may be determined by all methods known in the art, not limited to RT-PCR analysis and immunoassays.

In particular embodiments, the neoplastic disease is selected from the group consisting of hematologic cancer, such as a hematologic cancer selected from the group consisting of leukaemia (e.g. acute myeloid leukaemia (AML) or T cell acute lymphoblastic leukaemia (T-ALL), lymphoma, and myeloma (e.g. multiple myeloma), or a solid tumour, such as ovarian cancer, mesothelioma, melanoma, pancreas cancer, lung cancer, colon cancer, prostate cancer, breast cancer, head or neck cancer, kidney cancer or liver cancer)..

The invention also provides the polypeptides envisaged herein, or the pharmaceutical compositions comprising the polypeptides envisaged herein for use in the prevention and/or treatment of an autoimmune disease.
The term 'autoimmune disease' as used herein refers to a condition arising from and directed against an individual's own tissues or a co-segregate or manifestation thereof or resulting condition therefrom.
In particular embodiments, the MCL-1-mediated disease or disorder is selected from the list consisting of psoriasis, multiple sclerosis, inflammatory bowel disease, Hashimoto's thyroiditis, rheumatoid arthritis, ankylosing spondylitis, systemic lupus erythematosus, myasthenia gravis, renal inflammation and renal autoimmunity and severe allergic asthma.
In particular embodiments, the autoimmune disease is a B-cell mediated autoimmune disease. In particular embodiments, the autoimmune disease is selected from the list consisting of multiple sclerosis, rheumatoid arthritis, and systemic lupus erythematosus.
In particular embodiments, the subject or patient to be treated is a mammal, and more in particular a human suffering from, or at risk of a MCL-1- disease and disorder, such as a neoplastic disease, preferably a hematologic cancer, or an autoimmune disease.

The efficacy of the Alphabodies and polypeptides of the invention, and of compositions comprising the same, can be tested using any suitable *in vitro* assay, cell-based assay, *in vivo* assay and/or animal model known per se, or any combination thereof, depending on the specific disease or disorder involved. Suitable assays and animal models will be clear to the skilled person. For example the efficacy of an anti-MCL-1 Alphabody or Alphabody polypeptide can be easily determined in an *in vitro* assay, such as by cell death assays, apoptosis assays, such as cytochrome c release assays or flow cytometry assays (e.g. for detecting changes in the plasma and mitochondrial membranes, caspase activity, nuclear condensation, and fragmentation), or may be determined by BAK activation assays using antibodies specifically recognizing activated BAK.

Also envisaged herein is the use of the polypeptides directed against MCL-1 for diagnostic application. The polypeptides herein can be used in methods for determining aberrant expression and/or functioning of MCL-1. In particular embodiments this can be in the context of the diagnosis of a specific disease or disorder such as but not limited to cancers, autoimmune diseases, xxx.
In particular embodiments, methods are envisaged for determining the suitability of a particular treatment of a patient suffering from a disease or disorder such as, but not limited to diseases and disorders listed above.
The application also envisages the use of the polypeptides described herein in methods of screening compositions suitable for the treatment of diseases or disorders such as those described above.
Thus, also envisaged herein are kits comprising the polypeptides envisaged herein for use in a diagnostic or a screening method.

The application also envisages parts, fragments, analogs, mutants, variants, and/or derivatives of the polypeptides described herein comprising or essentially consisting of one or more of such parts, fragments, analogs, mutants, variants, and/or derivatives, as long as these parts, fragments, analogs, mutants, variants, and/or derivatives are suitable for the prophylactic, therapeutic and/or diagnostic purposes envisaged herein.

Such parts, fragments, analogs, mutants, variants, and/or derivatives are still capable of specifically binding to MCL-1.

Also provided herein are nucleic acid sequences encoding single-chain Alphabodies or Alphabody polypeptides, which are obtainable by the methods according to the invention as well as vectors and host cells comprising such nucleic acid sequences.
In a further aspect, the present invention provides nucleic acid sequences encoding the Alphabodies or the polypeptides of the invention (or suitable fragments thereof). These nucleic acid sequences are also referred to herein as nucleic acid sequences of the invention and can also be in the form of a vector or a genetic construct or polynucleotide. The nucleic acid sequences of the invention may be synthetic or semi-synthetic sequences, nucleotide sequences that have been isolated from a library (and in particular, an expression library), nucleotide sequences that have been prepared by PCR using overlapping primers, or nucleotide sequences that have been prepared using techniques for DNA synthesis known per se.
The genetic constructs of the invention may be DNA or RNA, and are preferably double-stranded DNA. The genetic constructs of the invention may also be in a form suitable for transformation of the intended host cell or host organism in a form suitable for integration into the genomic DNA of the intended host cell or in a form suitable for independent replication, maintenance and/or inheritance in the intended host organism. For instance, the genetic constructs of the invention may be in the form of a vector, such as for example a plasmid, cosmid, YAC, a viral vector or transposon. In particular, the vector may be an expression vector, i.e., a vector that can provide for expression in vitro and/or in vivo (e.g. in a suitable host cell, host organism and/or expression system). The genetic constructs of the invention may comprise a suitable leader sequence to direct the expressed Alphabody to an intended intracellular or extracellular compartment. For example, the genetic constructs of the invention may be inserted in a suitable vector at a pelB leader sequence site to direct the expressed Alphabody to the bacterial periplasmic space. Also the vector may be equipped with a suitable promoter system to, for example, optimize the yield of the Alphabody.
The application also provides vectors and host cells comprising nucleic acids described above. Suitable examples of hosts or host cells for expression of the Alphabodies or polypeptides of the invention will be clear to the skilled person and include any suitable eukaryotic or prokaryotic cell (e.g., bacterial cells such as E. coli, yeast cells, mammalian cells, avian cells, amphibian cells, plant cells, fish cells, and insect cells), whether located in vitro or in vivo.

The production of the polypeptides envisaged herein may comprise the step of expressing a nucleotide sequence encoding said polypeptide in a host organism under suitable conditions, so as to produce said polypeptide. This step can be performed by methods known to the person skilled in the art.
In addition, the polypeptides envisaged herein may be synthesized as soluble protein construct, optionally after their sequence has been identified by, for instance, in silico modelling.
For instance, the polypeptides envisaged herein can be synthesized using recombinant or chemical synthesis methods known in the art. Also, the polypeptides envisaged herein can be produced by genetic engineering techniques. Thus, for an Alphabody capable of binding to MCL-1, methods for synthesizing the polypeptides envisaged herein may comprise transforming or infecting a host cell with a nucleic acid or a vector encoding a polypeptide sequence having detectable binding affinity for, or detectable *in vitro* activity on, MCL-1 and having detectable binding affinity for albumin, preferably human serum albumin. Accordingly, the polypeptide sequences having detectable binding affinity for, or detectable *in vitro* activity on, MCL-1 and having detectable binding affinity for albumin, preferably human serum albumin, can be made by recombinant DNA methods. DNA encoding the polypeptides can be readily synthesized using conventional procedures. Once prepared, the DNA can be introduced into expression vectors, which can then be transformed or transfected into host cells such as *E*. *coli* or any suitable expression system, in order to obtain the expression of polypeptides in the recombinant host cells and/or in the medium in which these recombinant host cells reside.
It should be understood, as known by someone skilled in the art of protein expression and purification, that the polypeptide produced from an expression vector using a suitable expression system may be tagged (typically at the N-terminal or C-terminal end of the Alphabody) with e.g. a Histidine or other sequence tag for easy purification.
Transformation or transfection of nucleic acids or vectors into host cells may be accomplished by a variety of means known to the person skilled in the art including calcium phosphate-DNA coprecipitation, DEAE-dextran-mediated transfection, polybrene-mediated transfection, electroporation, microinjection, liposome fusion, lipofection, protoplast fusion, retroviral infection, and biolistics.

Suitable host cells for the expression of the desired polypeptides may be any eukaryotic or prokaryotic cell (e.g., bacterial cells such as *E*. *coli,* yeast cells, mammalian cells, avian cells, amphibian cells, plant cells, fish cells, and insect cells), whether located *in vitro* or *in vivo.* For example, host cells may be located in a transgenic animal.
Thus, the methods for the production of polypeptides herein are also provided, comprising transforming, transfecting or infecting a host cell with nucleic acid sequences or vectors encoding such polypeptides and expressing the polypeptides under suitable conditions.

Methods of producing Alphabodies or Aphabody polypeptides including the generation and screening of a random library of Alphabody polypeptides is known in the art, for example as described in published international patent application WO 2014/064092 in the name of Complix NV. In an alternative embodiment, it is envisaged that for the production of target-specific polypeptides, i.e. having detectable binding affinity for, or inhibitory activity on intracellular target molecules, binding sites can be introduced on an Alphabody structure sequence based on mimicry.
The process of producing Alphabodies or Alphabody polypeptides based on the process of mimicry is disclosed in detail in published international patent application WO2012/093013 in the name of Complix NV.
Where the Alphabody peptide is intended to bind to MCL-1, particular screening steps can further be envisaged. In particular embodiments, the methods of production comprise a step of determining whether the polypeptide binds to MCL-1 with a dissociation constant (KD) of less than about 1 nanomolar (1 nM), preferably less than about 10 picomolar (10 pM). Preferably the method comprises determining whether said binding affinity for MCL-1 is equal to or better than the binding affinity of a polypeptide comprising, consisting essentially of or consisting of an amino acid sequence as set forth in SEQ ID NO: 27.
The inventors have demonstrated that albumin binding region as exemplified herein may be successfully introduced into a polypeptide comprising at least one Alphabody structure capable of specifically binding to a target, such as MCL-1, by docking the alpha-helix of the *F. magna* protein G onto the HRS3 of the Alphabody, followed by grafting the residues not important for Alphabody folding (including the second and third helices of the *F. magna* protein G) taking into account potential interaction with the binding of the target. More particularly the inventors have found that optimal compatibility with the Alphabody backbone can be achieved by introducing an albumin binding region conjugated to the C-terminus of HRS3 of the Alphabody structure, wherein said albumin binding region comprises a sequence SDFYFXXINKA (SEQ ID NO: 2; also referred to herein as the fourth alpha-helix of the polypeptide as envisaged herein or as alpha-helix D) and a sequence TXEXVXALKXXILXAH (SEQ ID NO: 3; also referred to herein as the fifth alpha-helix of the polypeptide as envisaged herein or as alpha-helix E), wherein X can be any amino acid.
In particular embodiments, the methods of production comprise a step of determining whether the polypeptide binds to albumin, preferably human serum albumin, with a dissociation constant (KD) of less than about 20 micromolar (10 µM), preferably less than about 10 micromolar (10 µM), more preferably less than about 1 micromolar (1µM). Preferably the method comprises determining whether said binding affinity for albumin is equal to or better than the binding affinity of a polypeptide comprising, consisting essentially of or consisting of an amino acid sequence as set forth in SEQ ID NO: 27.

The inventors have further identified methods for introducing motifs for facilitating cellular entry of an Alphabody. Accordingly the application provides a method for producing a polypeptide comprising at least one alphabody capable of specifically binding to an intracellular target, which comprises conjugating the Alphabody or polypeptide comprising at least one Alphabody structure and an albumin binding region to at least one, such as one or two, peptide(s) for facilitating cellular entry of the polypeptide, wherein said peptide(s) comprise(s), consist(s) essentially of or consist(s) of, a sequence (RP)ₙ, wherein n is an integer from 6 to 9, such as 6, 7, 8 or 9, preferably 7, 8 or 9, more preferably 7. In particular embodiments the methods of production comprise the step of determining whether the polypeptide is capable of entering the cell and/or binding to an intracellular target. Such methods are known in the art and illustrated herein.

The inventors have moreover found that the provision of an albumin binding region and a peptide for facilitating cellular entry (e.g. cellular uptake sequence motif) as described herein provides optimal stability without compromising the ability to enter into the cell. Accordingly in particular embodiments, the provision of a polypeptide comprising an Alphabody against an intracellular target encompasses both aspects.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and scope of the appended claims.

The herein disclosed aspects and embodiments of the invention are further supported by the following non-limiting examples.

### EXAMPLES

### Example 1. Materials and methods for examples 2 to 7

The term "Alphabody" can be used in the example section to refer to the Alphabody structure *per se* or the polypeptide comprising the at least one Alphabody structure.

### Expression and purification of Alphabodies

Alphabodies were recombinantly produced as reported previously (J. Desmet, K. Verstraete, Y. Bloch, E. Lorent, Y. Wen, B. Devreese, K. Vandenbroucke, S. Loverix, T. Hettmann, S. Deroo, K. Somers, P. Henderikx, I. Lasters, S. N. Savvides, Structural basis of IL-23 antagonism by an Alphabody protein scaffold, Nat. Commun. 5, 5237 (2014)). Briefly, recombinant production of Alphabodies with an N-terminal decahistidine tag was performed in BL21(DE3)pLysS cells (Life Technologies), grown in Luria-Bertani medium at 37 °C. At an OD600 nm of 0.6, expression was induced by addition of 1 mM isopropylthio-β-galactoside. Expression was allowed for 4 h at 37 °C, after which cells were harvested by centrifugation, resuspended in Tris buffer and frozen at -80 °C. Cell lysis was performed by thawing and sonication in presence of DNAseI and MgCl₂, after which the suspension was cleared by centrifugation at 40 000 rpm. Alphabodies were isolated from the soluble fraction using a 5-ml IMAC HP Ni sepharose column (GE Healthcare), followed by a polishing and desalting step, performed on a Superdex 75 size-exclusion column (GE Healthcare) equilibrated in phosphate buffered saline (PBS).

### Production and purification of recombinant human MCL-1

The reference sequences of the cDNA encoding for MCL-1 residues Asp₁₇₂-Gly₃₂₇ (_{ΔNΔC}MCL-1) (NM_001197320.1) were synthesized by Geneart as a co-translational fusion with Glutathione S-transferase and cloned in the pET42 vector (Novagen). The GST-MCL-1 fusion protein was expressed in BL21(DE3)pLysS cells (Life Technologies). Cultures were grown at 37 °C in Luria broth supplemented with 100 µg/mL kanamycin. At an OD600 nm of 0.6, expression was induced by addition of 1 mM isopropylthio-β-galactoside. Expression was allowed for 4 h at 30 °C, after which cells were harvested by centrifugation, resuspended in PBS buffer and frozen at -80 °C. Cell lysis was performed by thawing and sonication in presence of DNAseI, MgCl₂ and 4-(2-aminoethyl)benzenesulfonyl fluoride hydrochloride (AEBSF), after which the suspension was cleared by centrifugation at 40 000 rpm. Purification of the fusion construct was performed from the soluble fraction using glutathione S-transferase (GST) Trap FF column (GE Healthcare), followed by a polishing step performed on a Superdex 75 size-exclusion column (GE Healthcare) equilibrated in Tris buffer.

### Solution ELISA assays for MCL-1 affinity determination

A Nunc plate was coated with 1 µg/mL anti-M13 (Fischer Scientific) in PBS for 2 h at room temperature (RT). The plate was then blocked with 2% skimmed milk in PBS for 2 h at RT while shaking. A proprietary Alphabody-displaying phage with high affinity for MCL-1 was added in PBS + 1% (v/v) bovine serum albumin (BSA) for 1 h at RT while shaking. In parallel with previous steps, a mixture of 50 pM GST-tagged MCL-1 and a dilution series of test Alphabody (seven 2-fold dilutions starting at 2 nM plus a blank) in PBS + 1% BSA was preincubated in a deep-well plate for 4 h at RT. In addition, a 'calibrator' dilution series of GST-tagged MCL-1 (seven 2-fold dilutions starting at 100 pM plus a blank) in PBS + 1% BSA was prepared. The MCL-1-Alphabody mixtures and the calibrator series were then transferred to the enzyme-linked immunosorbent assay (ELISA) plate in duplicate (two columns for each series) and incubated for another 1 h at RT while shaking. Detection of bound GST-MCL-1 was performed with 1/5000 goat anti-GST-horseradish peroxidase (HRP) (Abcam) in PBS + 1% BSA for 30 min at RT while shaking. After each incubation step, the plate was washed 4x with PBS containing 0.05% Tween 20, except after the incubation step of the mixture and calibrator samples where the plate was washed 12 times. The plate was developed with SureBlue and the reaction was stopped with H₂SO₄. Optical densities (ODs) were read at 450 nm. For the calculation of Alphabody-MCL-1 affinity, the OD signals from the calibrator series were first fitted to a sigmoid function, the latter providing the relationship between the measured ODs and free GST-MCL-1. Using this relationship, the OD values measured for the Alphabody-MCL-1 mixtures were converted to free GST-MCL-1 concentrations, and the latter were then fitted to the quadratic equation for Alphabody-MCL-1 binding, using the solution KD as parameter.

### Solution ELISA assays for albumin affinity determination

An ELISA assay was elaborated for CPAB-albumin affinity determination exploiting the observation that CPABs show affinity for skimmed milk-blocked Nunc plates, but not when bound to albumin. A Nunc plate was blocked O/N with PBS + 2% skimmed milk in PBS at 4 °C. A mixture of 8 nM CPAB (CMPX-383B or -359E) and dilution series of HSA, RSA or MSA were prepared (seven 3-fold dilutions starting at 10 µM plus a blank) in 100 mM TRIS + 100 mM NaCl + 1% BSA at pH 7.4 in deep well plates and preincubated for 30 min at RT while shaking. In addition, a 'calibrator' dilution series of CPAB (seven 3-fold dilutions starting at 100 nM plus a blank) in 100 mM TRIS + 100 mM NaCl + 1% BSA at pH 7.4 was prepared. The albumin-CPAB mixtures and the calibrator series were then transferred to the ELISA plate in duplicate (two columns for each series) and incubated for another 30 min at RT while shaking. Detection of plate-bound CPAB was performed with 1/5000 anti-V5-HRP (Invitrogen, R961-25) in PBS + 0.1% BSA for 30 min at RT while shaking. After each incubation step, the plate was washed 4x with PBS containing 0.05% Tween 20. All further steps, including the conversion of OD values into free CPAB concentrations using the calibrator series, and the fitting of solution KDs, were the same as for MCL-1 affinity determination.

### Circular dichroism experiments

Prior to the measurement, Alphabodies, stored in 20 mM HEPES pH 7.2 and 150 mM NaCl, were centrifuged and diluted to 200-340 µg/ml in PBS with indicated concentrations of Guanidine hydrochloride. The fraction of α-helical content was calculated from the molar residue ellipticity (MRE) at 222 nm. For determination of the thermal stability, the up scan (5 °C to 95 °C) was immediately followed by a down scan (95 °C to 5 °C). The reported T_{M} value was calculated as the average temperature of the 2 minima. Reported values for reversibility of folding were calculated as the ratio of MRE at 25 °C from the down scan vs the up scan.

### Western blot experiments probing for cellular uptake, followed by calculation of cytosolic Alphabody concentrations.

NCI-H929 cells were seeded in 12-well plates (Greiner Bio-One, # 665180) at a density 6x10⁵ cells per well in standard culture medium. After overnight growth, either Alphabody CMPX-321A, either the non-CPAB formatted Alphabody MB23, were added to a final concentration of 2 µM and incubated for either 2 h or 6 h at 37 °C. After incubation, the cells were harvested, the cell content was fractionized into a cytosolic and rest fraction, (Abcam Cytosol/Particulate Rapid Separation Kit, # ab65398) and loaded on SDS-PAGE (10% Criterion XT Bis-Tris Gel, Bio-Rad) together with a dilution series of CMPX-321A. Protein content was electroblotted to a 0.2 µM polyvinylideenfluroide (PVDF) membrane (Trans-Blot Turbo Midi 0.2 µm PVDF, Bio-Rad). After blocking and washing, Alphabodies were detected using a mix of antibodies (Anti-Histidine Tagged Antibody clone HIS.H8, Merck # 05-949, 1/2000 diluted and supplemented with 2 proprietary monoclonal antibodies recognizing a common epitope in the α-helix B), and visualized using an anti-mouse alkaline phosphatase (AP) secondary antibody (Anti-Mouse IgG AP Conjugate, Promega # S3728, 1/5000 diluted). The calculation of cytosolic concentration is based on in-house confocal microscopy experiments. These allowed to estimate the volume an NCI-H929 cell to be approximately 1 pL. The intensity of the CMPX-321A signal in the cytosolic fraction of Figure 2b was higher than the 17 µM (10 pmol) calibrator lane. Given that the isolated cytosolic fraction of NCI-H929 cells is even lower than the total cell volume (in which other organelles, such as the relatively big nucleus, are present), present inventors can infer that the intracellular concentration of CMPX-321A exceeds 17 µM as well.

### Immunofluorescence experiments on HELA cells

Hela cells were seeded in 8-well Nunc^{™} Lab-Tek^{™} Chamber Slides (Thermo Fisher Scientific, #177402), treated with 1 µM MCL1 targeting CPAB in the presence or the absence of 10 µM mouse serum albumin (MSA) for the indicated time points. Cells were fixed and permeabilized with 100% methanol and blocked with 2% bovine serum albumin (BSA) in DPBS with 0.05% Triton x-100 blocking (1 hour at RT), before incubating with anti-V5 Tag Monoclonal (1 hour at RT; Thermo Fisher Scientific, #R960-25). Primary antibody was visualized using anti-mouse Alexa Fluor 488 conjugated (45min at RT; Thermo Fisher Scientific, #A-21202). Vybran DyeCycle Ruby Stain (30min at RT; Thermo Fisher Scientific, #10115414) was used to dye the nuclei. Chambers were removed and coverslips mounted with Fluorescence Mounting Medium (Dako #S3023). Images were obtained using Zeiss LSM 510 META confocal microscope and analyzed using Fiji - ImageJ.

### In vitro treatment of MM cell lines with CMPX-321A

Human myeloma cell lines (HMCLs) were derived from primary myeloma cells cultured in RPMI 1640 medium supplemented with 5% fetal calf serum and 3 ng/mL recombinant IL-6 for IL-6 dependent cell lines. Cell viability was measured using CellTiter-Glo Luminescent Cell Viability Assay from Promega. As control and to calculate percentages of cell viability, at least triplicates of cells in medium only (without test compound) were included on every plate. Luminescence signal of these wells (cells without test compound) corresponds to 100% cell viability. The start concentration of CMPX-321A and CMPX-322M was 5 µM with a two-fold dilution step and 7 concentration points (5 - 2.5 - 1.25 - 0.625 - 0.313 - 0.156 - 0.078 µM). As buffer control, dilutions of buffer corresponding to the 7 dilutions of test compounds were included. All experiments were repeated 3 times and each experimental condition, including the buffer control, was repeated in duplicate wells in each experiment. The inhibition effect was calculated after 72h incubation with the following formula: % of viability = (Luminescence of treated cells/Luminescence of cells with medium only) X 100. IC₅₀ values were calculated using the GraphPad Prism Software and a 4 parameters non-linear regression with 100% cell survival in medium as top constraint and 0% cell survival as bottom constraint.

### Analysis of myeloma cell lines sensitivity to CMPX-321A in function of MCL-1 gene expression

The gene expression of MCL-1 was obtained from the gene expression profile database at Myelomax. HMCLs RNA were prepared from exponentially growing cells. RNA was extracted using the RNeasy Kit (Qiagen, Hilden, Germany). Biotinylated cRNA was amplified with a double in vitro transcription and hybridized to the human U133 2.0 plus GeneChips, according to the manufacturer's instructions (Affymetrix, Santa Clara, CA, USA). Fluorescence intensities were quantified and analyzed using the GECOS software (Affymetrix). Gene expression data were normalized with the MAS5 algorithm. The database contains the gene expression profile of the 33 parental MM cell lines. Several probes are available for each gene and for the analysis, a probe for the MCL-1 gene (MCL-1 probe 200797_s_at) was selected based on the major variation in expression level.

### Co-immunoprecipitation of MCL-1:BAK and MCL-1:BIM complexes in presence of Alphabodies

NCI-H929 cells were treated with the Alphabodies CMPX-321A or control CMPX-322M for two or six hours at a concentration of 0.45 µM. A control condition with no Alphabody treatment was included. After washing cells with PBS, cell pellets were resuspended in lysis buffer (25mM Tris-HCl pH7.4; 150 mM NaCl; 0.1% NP40; 2.5 mM MgCl₂, 5% glycerol) and precleared with protein A. Primary Antibody (anti- MCL-1 Ab from Santa Cruz; S19) was first bound to Dynabeads Protein A. Beads and cell lysis sample were mixed for 10 minutes at room temperature. Immunocomplexes captured on the Dynabeads were washed and denatured. Lysates of total protein and the immunoprecipitates were separated by SDS-PAGE, electrotransferred to polyvinylidene difluoride membranes, and analyzed for the presence of MCL-1 (anti- MCL-1 Ab from Santa Cruz; S19 and MCL-1 Monoclonal Antibody (RC13) from Invitrogen; AH00102), BAK (556382, BD Biosciences), Bim (AB17003, Merck Millipore) and Alphabodies. Alphabodies are detected by using a mixture of mouse monoclonal anti-His antibody (1/2000), Complix' mouse monoclonal anti-Alphabody antibodies 1D6F5 (2 µg/ml) and 4D8E5 (2 (µg/ml). Protein detection was performed with the Chemidoc MP Imager and the ImageLab software.

### BAK activation assay

NCI-H929 cells were treated with two-fold dilutions starting at 10 µM of CPABs in 500 µl complete cell culture medium containing 10% fetal bovine serum. Cells were incubated for 4h at 37 °C. After CPAB treatment, cells were washed with PBS and permeabilized using the Cytofix/Cytoperm^{™} Fixation/Permeabilization Solution Kit (BD Biosciences) following manufacturer's recommendations. Activated BAK was detected with the primary anti-BAK antibody TC-100 (Merck Millipore). After a washing step, detection of the primary anti-BAK antibody was performed with an AlexaFluor488 labeled secondary anti-mouse antibody. To exclude dead cells from data analysis the LIVE/DEAD^{™} Fixable Near-IR Dead Cell Stain (Invitrogen) was included. Finally, cells were washed once and measured on a BD LSR Fortessa cell analyzer. The flow cytometry data was analyzed using either FACSDIVA or FlowJo software.

### CMPX-383B test item handling for in vivo experiments

The vials containing the Alphabody CMPX-383B were stored at -80 °C until the day of use. On the day of use, CMPX-383B was thawed for 30 minutes in a water bath at 37 °C. Before use, the test substance was visually inspected for sign of turbidity. If turbidity was observed, the vial of the test substance was put in the water bath at 37 °C for another 15 minutes. The test substance was used within 1 hours of thawing for all *in vivo* experiments.

### In vivo treatment of MM xenograft models

A MOLP-8 subcutaneous xenograft model was established in 20 nonobese diabetic/severe combined immunodeficient γ (NSG) female mice at the age of 6-8 weeks by injecting 1x10⁷ in human multiple myeloma MOLP-8 cells at the right flank region. Before tumor cell inoculation, each animal was pretreated for 2 days with a daily intraperitoneal injection of 150mg/kg cyclophosphamide to facilitate engraftment of the MOLP-8 cells. Once the tumors reached an average volume of 80 mm³ (range between 50-100 mm³), mice were randomized in two groups and either treated daily with vehicle (20mM HEPES pH 7.2 150mM NaCl) or CMPX-383B (20mg/kg body weight) for 14 consecutive days. Tumor volume was measured twice a week using a caliper until 20 days after the last treatment administration. Mice were closely monitored and killed if tumor volume reaches 3000mm³ or any humane end points. For the NCI-H929 subcutaneous xenograft model 20 female CB-17 Scid mice (nomenclature: CB-17/Icr-Prkdcscid/Rj) at the age of 8 weeks were subcutaneously inoculated with 5x10⁶ NCI-H929 tumor cells into their right flank. Tumor volume was measured twice a week by caliper and body weight was determined in parallel. Once an average tumor volume of 80 mm³ was reached, animals were randomized into 2 groups. The animals received once daily, repeated intravenous doses of CMPX-383B (20 mg/kg) or vehicle (20mM HEPES pH 7.2 150mM NaCl).

### In vivo treatment of a T-ALL xenograft with CMPX-383B

A xenograft of primary human T-ALL cells from patient 8 was established in 16 nonobese diabetic/severe combined immunodeficient γ (NSG) female mice at 16 weeks of age by injecting 1.5×10⁶ patient derived T-ALL cells in 150 µL phosphate-buffered saline through tail vein injection. The engraftment of these leukemic cells was monitored at regular time points by human CD45 staining (CD45-FITC antibody; Miltenyi Biotec) in the peripheral blood using FACS analysis with BD LSR II (BD Biosciences). Upon detection of human CD45+ leukemic blasts in peripheral blood, mice were randomized in 2 groups and treated with either vehicle (20mM HEPES pH 7.2 150mM NaCl) or CMPX-383B. During treatment, the percentage of human CD45-positive leukemic blasts in the peripheral blood was determined at regular time points. In the first week of treatment, both vehicle and CMPX-383B (25mg/kg body weight) were administered intravenously in the tail vein for 7 consecutive days in the morning. During the second week, present inventors wanted to maximize the effect of CMPX-383B and an extra dose of CMPX-383B (25mg/kg body weight) or vehicle was administered for 7 consecutive days in the evening via IP injection, on top of the daily dose of CMPX-383B via tail vein administration as previously described. The control mice were closely monitored and checked for signs of leukemia or loss in body weight (<20% of start body weight). After the last treatment, the end percentage of human CD45-positive leukemic blasts in the blood from both vehicle and CMPX-383B mice were analyzed via CD45 FACS staining as previously described and all animals were sacrificed. The ethical committee on animal welfare at Ghent University Hospital approved this animal experiment.

### Statistical analysis of in vivo tumor evolution in xenograft models

Special consideration was given to the statistical analysis of the acquired data. Recognizing the presence of correlated observations (i.e. the response is measured repeatedly on a set of mice), present inventors analyzed tumor volumes as longitudinal data, also known as repeated measurements. The defining feature of longitudinal data is the possibility to characterize changes over time (e.g. tumor progression), and factors that influence that change. This is in contrast to the more commonly performed analysis at each time point, which only provides information about the differences in treatment effects at that specific time point, and, hence, does not consider the evolution in treatment effects. Ordinary linear regression can model the evolution in outcome over time but does not accommodate for the correlation between observations. Therefore, in repeated measurements analysis, the aim is not only to characterize changes over time and factors that influence that change, but also to model the correlation across time within subjects, in order to get better estimates of treatment effects and the error. Present inventors analyzed tumor volumes and hCD45 percentages as repeated measurements using method of residual maximum likelihood (REML), as implemented in Genstat version 20. Briefly, a linear mixed model (random terms underlined) of the form y = treatment + time + treatment × time + mouse × time was fitted to the longitudinal data. The term mouse × time represents the residual error term with dependent errors because the measurements are taken in the same individual, causing correlations among observations. Several covariance models were fitted to the data to account for the correlation present in the data. The autoregressive correlation model of order 1 (AR1) with times of measurement set at equal intervals, was selected as best fitted model based on the Akaike's information criterion coefficient (AIC), for analysis of tumor volumes. The AR covariance model assumes that correlation between observations decays as the measurements are collected further apart in time. The first order refers to the fact that only recent (t-1) observations affect the current observations made at timepoint t. The antedependence correlation model of order 1 (ANTE1) with times of measurement set at unequal intervals, was selected as best fitted model based on the AIC, for analysis of hCD45 percentages. The ANTE 1 covariance model assumes that correlation between observations decays as the measurements are collected further apart in time, just like the AR covariance model does, but allows for changes in correlation structure over time. In addition, the ANTE covariance model allows for unequal variances across time. The significance of the fixed terms in the mixed model were assessed using an approximate F-test as implemented in Genstat version 20.

### MCL-1 mRNA expression level in a panel of primary T-ALL patients

Total RNA was isolated using the RNeasy Plus Micro Kit (Qiagen). The iScript cDNA synthesis kit (Bio-Rad) was used to synthesize cDNA. The quantitative real-time polymerase chain reactions were performed using the SsoAdvanced SYBR Green Supermix (Bio-Rad) and were run on the LightCycler 480 (Roche, model LC480). Every sample was analyzed in duplicate and MCL-1 gene expression was standardized against at least 3 housekeeping genes. The primer sequences are as follows:
MCL-1 Fw 5'-GTCCTCTACATGGAAGAACTC-3' (SEQ ID NO: 28)
MCL-1 Rv 3'-ACACTTGAAGACCATAAACCA-5' (SEQ ID NO: 29)
UBC_Fw 5'-ATTTGGTGCGCGGTTCTTG-3' (SEQ ID NO: 30)
UBC_Rv 3'-TGCCTTGACATTCTCGATGGT-5' (SEQ ID NO: 31)
TBP_Fw 5'-CACGAACCACGGCACTGATT-3' (SEQ ID NO: 32)
TBP_Rv 3'-TTTTCTTGCTGCCAGTCTGGAC-5' (SEQ ID NO: 33)
HPRT1_Fw 5'-TGACACTGGCAAAACAATGCA-3' (SEQ ID NO: 34)
HPRT1_Rv 3'-GGTCCTTTTCACCAGCAAGCT-5' (SEQ ID NO: 35)

### Expression and purification of recombinant protein complexes for crystallization.

The reference sequences of the cDNA encoding for MCL-1 residues Asp₁₇₂-Gly₃₂₇ (_{ΔNΔC}MCL-1) (NM_001197320.1) were synthesized by Geneart and were a kind gift from Complix NV. A Glutathione-S-Transferase (GST) fusion construct was created in which _{ΔNΔC}MCL-1 was cotranslationally fused to GST, separated by a thrombin recognition sequence. The fusion construct was cloned in the pET15b vector (Novagen) using NcoI and BamHI restriction sites. In parallel, a construct was created in which for crystallography optimized variants of CMPX-383B, lacking the CPAB sequences, could be expressed in the pET15b vector with a thrombin-removable hexahistine sequence. For isolation of the _{ΔNΔC}MCL- 1:Alphabody complex, E. coli BL21(DE3) strains were transformed with the expression construct of either GST- _{ΔNΔC}MCL-1, either the Alphabody. Single colonies of both plates were used to inoculate 10 ml LB medium for overnight growth. Next day, both precultures were used to inoculate 31 LB medium per construct. At OD600 nm of 0.6, expression was induced with 1mM IPTG final concentration. GST-_{ΔNΔC}MCL-1 was expressed for 4 h at 28 °C, after which the bacteria were harvested by centrifugation (6000 rpm for 15 min at 4 °C) and the cellular paste was stored at -80 °C. Expression of the Alphabody was continued overnight. Next day, the bacteria expressing the Alphabody were harvested, whilst the bacterial pellet with GST-_{ΔNΔC}MCL-1 was thawed. Both pellets were resuspended in 45 ml phosphate buffer (300 mM NaH₂PO₄ pH 7.0, 300 mM NaCl) per liter expression culture, and mixed together prior to cell lysis. The cells were lysed by sonication on a Q500-220 QSonic sonicator (run time of 4 minutes with 30 s pulses at 80% output interspersed with 30 s of down time) and centrifuged for 30 min at 20 000 rpm. The supernatant of both lysates was collected, filtrated through a 0.22 µm filter and mixed. This mixture was then finally applied overnight on two 1 ml GSTrap (GE Healthcare) at a flow speed of 0.1 ml/min. Next day, both columns were extensively washed with phosphate buffer, after which 6 units of biotinylated thrombin (Novagen) was injected. After injection, the columns were detached and incubated overnight at room temperature. Next day, elution of thrombin-cleaved _{ΔNΔC}MCL-1, alone or in complex with complex with thrombin-cleaved Alphabody, was performed by restoring the flow on the GSTrap columns. _{ΔNΔC}MCL-1:CMPX-383B was finally separated from thrombin and _{ΔNΔC}MCL-1 alone using Size Exclusion Chromatography (Superdex 75 HR 10/30 column, GE Healthcare), with 20 mM HEPES pH 7.4 and 150 mM NaCl as equilibration buffer. For isolation of Alphabody:HSA complexes, the Alphabody was expressed as described above. Subsequent to cell lysis, his-tagged Alphabody was captured using Immobilized Metal Affinity Chromatography (5ml HisTrap FF prepacked column, GE Healthcare) and polished using SEC (Superdex 75 HR 10/30 column, GE Healthcare). The complex with human serum albumin (Sigma) was assembled and purified via SEC.

### Crystal structure determination of the _{ΔNΔC}MCL-1: CMPX-383B complex

_{ΔNΔC}MCL-1:CMPX-383B was concentrated to 5.1 mg/ml. Commercial sparse matrix crystallization screens (Molecular Dimensions, Hampton Research, Jena Bioscience) were set up in a vapor-diffusion fashion, by mixing 100 nL protein solution with 100 nL mother liquor in SwissSci 96-well triple drop plates. Plates were incubated at 294 K. After 6 months, crystals were detected in condition C7 of the JCSG-plus crystal screen (Jena Bioscience), containing 0.2 M zinc acetate dihydrate, 0.1 M sodium acetate pH 4.5 and 10 % (w/v) PEG 3000. These hits could be further optimized using gradient optimization in 96-well format and at different temperatures. Crystals diffracting X-rays to 2.3 Å were grown at 287 K in a condition containing 0.2 M zinc acetate dihydrate, 0.1 M sodium acetate pH 5.5 and 2% PEG 3000. Prior to cryo-cooling by plunging into liquid nitrogen, these crystals were step-wise cryoprotected to contain a final concentration of 30% (v/v) PEG 400. Diffraction data was collected at 100 K at the ESRF ID23-2 beamline, Grenoble. Diffraction data was indexed, integrated and scaled using the XDS suite (W. Kabsch, Integration, scaling, space-group assignment and post-refinement, Acta Crystallogr. Sect. D Biol. Crystallogr. 66, 133-144 (2010)). Initial phases were obtained using maximum-likelihood molecular replacement by Phaser from the CCP4 package (M. D. Winn, et al. Overview of the CCP4 suite and current developmentsActa Crystallogr. Sect. D Biol. Crystallogr. 67, 235-242 (2011); A. J. McCoy, et al., Phaser crystallographic software, J. Appl. Crystallogr. 40, 658-674 (2007)) using the crystallographic model of MCL-1 as a search model (PDB code 3MK8) (M. L. Stewart, et al., The MCL-1 BH3 helix is an exclusive MCL-1 inhibitor and apoptosis sensitizer., Nat. Chem. Biol. 6, 595-601 (2010)). Structure building and refinement was performed iteratively using COOT (P. Emsley, et al., Features and development of Coot, Acta Crystallogr. Sect. D Biol. Crystallogr. 66, 486-501 (2010)), PHENIX (P. D. Adams, et al., PHENIX: A comprehensive Python-based system for macromolecular structure solution, Acta Crystallogr. Sect. D Biol. Crystallogr. 66, 213-221 (2010)), BUSTER (G. Bricogne, et al. BUSTER version 2.10.3, Cambridge, United Kingdom Glob. Phasing Ltd. (2011)) and the PDB-REDO server (R. P. Joosten, et al., The PDB-REDO server for macromolecular structure model optimization, IUCrJ (2014), doi:10.1107/S2052252514009324). The Alphabody structure was built and chain-traced from scratch into the electron density.

### Crystal structure determination of the HSA:CMPX-383B complex

Purified HAS:CMPX-383B complex was concentrated to 95 mg/ml. Crystallization screens containing 14% - 24% PEG 3350, 0.2 M ammonium phosphate and 0.1 M of acetate (pH 4.2 - pH 5.6), BIS-TRIS (pH 5.8 - pH 7.0) or BIS-TRIS propane (pH 7.0 to pH 9.0) buffer were set up in a vapor-diffusion fashion, by mixing 100 nL protein solution with 100 nL mother liquor in SwissSci 96-well triple drop plates. Plates were incubated at 287 K, 294 K or 310 K. Crystals with a plate-like morphology, diffracting X-rays to 3.27 Å, were grown at 310 K in a condition containing 18% (w/v) PEG 3350, 0.1 M BIS-Tris pH 6.8 and 0.2 M ammonium phosphate. Prior to cryo-cooling by plunging into liquid nitrogen, these crystals were cryoprotected to contain a final concentration of 25% (w/v) PEG 3350 and 10% (v/v) glycerol. Diffraction data was collected at 100 K at the P14 microfocus beamline at PETRA III, Hamburg. Diffraction data was indexed, integrated and scaled using the XDS suite (W. Kabsch, Integration, scaling, space-group assignment and post-refinement, Acta Crystallogr. Sect. D Biol. Crystallogr. 66, 133-144 (2010)). The resulted dataset was truncated and rescaled using the STARANISO anisotropy & Bayesian estimation server (I. J. Tickle, et al., STARANISO (2018)). Initial phases were obtained using maximum-likelihood molecular replacement by Phaser from the CCP4 package using the crystallographic model of HSA as search model (PDB code 4Z69) (Y. Zhang, et al., Structural Basis of Non-Steroidal Anti-Inflammatory Drug Diclofenac Binding to Human Serum Albumin, Chem. Biol. Drug Des. (2015), doi: 10.1111/cbdd.12583). Structure building and refinement was performed iteratively using COOT, PHENIX, BUSTER and the PDB-REDO server. The Alphabody structure was built and chain-traced from scratch into the electron density.

### Example 2. Alphabody CMPX-152A integrates the MCL-1 BH3 binding principles into a designed protein scaffold

The Alphabody protein scaffold consists of a contiguous polypeptide that folds into a three-helix coiled-coil with antiparallel helix topology, and its entire scaffold surface can be deployed to generate a binding surface for a given target (Figure 1a). To explore the potential of the Alphabody platform for tailored binding to intracellular targets, present inventors sought to neutralize MCL-1, a member of the anti-apoptotic BCL-2 family and a clinically validated drug target. It has previously been described that the BH3 helix of MCL-1, when expressed as a standalone molecular entity, becomes a high-affinity inhibitor of MCL-1 (M. L. Stewart, et al., The MCL-1 BH3 helix is an exclusive MCL-1 inhibitor and apoptosis sensitizer., Nat. Chem. Biol. 6, 595-601 (2010)). Present inventors hypothesized that MCL-1-interacting residues could be engrafted onto one of the Alphabody α-helices to yield a designed Alphabody that can bind to MCL-1 with high affinity and specificity. Based on the available complexes of MCL-1 and different inhibitory peptides, present inventors selected 6 residues that were hypothesized to play a key role in the specific binding to MCL-1 (Figure 1b). Together, these residues were positioned to form an amphipathic helical element with hydrophobic residues (Leuₙ, Valₙ₊₃ and Valₙ₊₇) on one face and Argₙ₊₁/Aspₙ₊₅ projecting a polar helical face. In addition, Glyₙ₊₄ was identified as a key determinant for MCL-1 binding, as any residue with a sidechain larger than that of alanine would likely sterically impede MCL-1 binding. Present inventors engrafted these 6 amino acids onto the B-helix of the Alphabody scaffold to yield Alphabody CMPX-152A as a prototype. In silico docking experiments revealed that incorporation of the MCL-1 inhibitory footprint into the Alphabody template required grafting of the Valₙ₊₃Glyₙ₊₄Aspₙ₊₅ sequence on heptad positions bcd (Figure 1c, grey background and underlined). However, this resulted in the insertion of a negatively charged aspartate residue into the hydrophobic all-isoleucine core of the Alphabody, which would be expected to compromise the stability of the Alphabody. In the absence of options to engineer interactions that electrostatically stabilize this so-called "Asp-in-core", present inventors applied a negative design methodology that, per design, averted the Alphabody from adopting undesired conformations, such as possible oligomeric species or aggregates. To do so, present inventors further destabilized the Asp_{B3d}-containing core layer (Figure 1c, grey triangle in full lines) by substituting Ile_{A2a} by a threonine (Figure 1c, grey background and circled). The rationale for opting for a threonine residue for this purpose was twofold. First, the amphipathic nature of this residue would allow both a polar interaction with the core aspartate (Figure 1c, grey triangle in full lines) mediated by its hydroxyl group, while its hydrophobic methyl group would be able to interface with the adjacent all-isoleucine core layer (Figure 1c, grey triangle in striped lines). Secondly, threonine is the only naturally occurring amino acid that combines polarity with an isoleucine-like C_{β}-branched configuration, which was considered advantageous for replacing an Ile in the Alphabody core. Although the energetic penalty of the Asp-in-core would be worsened by this additional polar residue in the Alphabody core, in silico modeling revealed that conformations that do not cluster Asp_{B3d} and Thr_{A2a} into the same core layer (Figure 1c, grey triangle in full lines), would be even more unstable and thus more unlikely to form. As such, present inventors actively engineered against the formation of any other conformation than the desired one - indeed a true hallmark of negative design. Remarkably, the resulting Alphabody CMPX-152A showed ultra-high affinity for MCL-1, with a KD = 8 pM (Figure Id). Circular dichroism measurements confirmed that CMPX-152A contained the expected amount of helical secondary structure, yet as expected, the destabilization of a core layer in its fold induced a significant reduction in thermal stability as compared to the Alphabody template (T_{M} of 58 °C and 120 °C, respectively, in presence of 1 M GuHC1) (Figure 1e-f). However, with a higher than 90% reversible thermal stability in the presence of a chaotrope (Table 2), CMPX-152A is more thermostable than the average mesostable protein (T_{M} of 54 °C in absence of GuHCl).

**Table 2. Overview of the thermal stability of different Alphabodies**

| | **CMPX-152A** | **CMPX-321A** | | **CMPX-383B** |
|---|---|---|---|---|
| Concentration GuHCl | 1M | 0 M | 1M | 1M |
| T_{M} | 57.6 °C | 73.7 °C | 45.7 °C | 85.1 °C |
| Reversibility | 91.6% | 96.0% | 100.0% | 96.6% |
| Thermal stabilities of the Alphabodies were determined using circular dichroism thermal denaturation experiments in presence of indicated concentrations of guanidium hydro-chloride (GuHCl). For each experiment, T_{M} values were calculated as the mean of the upscan and downscan inflection points of the sigmoid | | | | |

### Example 3. Reformatting of MCL-1 specific Alphabodies to enable cell penetration and intracellular inhibition

Given the intracellular location of MCL-1, present inventors developed a second generation of MCL-1 inhibitory Alphabodies that can be taken up by cells, thereby addressing a fundamental challenge in the targeting of intracellular drug targets by protein therapeutics. The mechanism of how particular sequences endow peptides with cell-penetrating capacities is still under debate. Nevertheless, it has been recognized that naturally occurring cell-penetrating sequences, such as TAT, penetratin and the synthetic peptides cFΦR4, mediate cell penetration by the presentation of guanidium groups to the negatively charged phospholipid bilayer. Present inventors leveraged such insights to develop reformatted Alphabodies that can translocate across the cell membrane without compromising their target binding properties. The developed cell-penetrating Alphabody (CPAB) tag consists of 7 consecutive arginine-proline repeats ([RP]₇) that were engineered to be added co-translationally at the N- and C-terminus of the Alphabody polypeptide (Figure 2a). The rationale for the choice of a proline residue flanked by arginines, was twofold. Based on currently known cell-penetrating peptides, present inventors inferred that a proline residue would increase uptake by reducing the electrostatic repulsion between the protein backbone amides and the lipid bilayer. Furthermore, the hydrophobic nature of a proline residue was expected to enhance the efficiency of cell penetration. Alphabody CMPX-321A carrying CPAB tags at its N- and C-terminus (Figure 2a) was found to efficiently cross the mammalian cell membrane (Figure 2b-c). Specifically, present inventors evaluated this newly acquired property by incubating NCI-H929 cells with CMPX-321A, followed by probing their cytosolic content for the presence of the Alphabody using antibodies recognizing the B-helix of CMPX-321A (Figure 2b). After a 2 h incubation with 2 µM of added Alphabody, the detected signal for CMPX-321A in the cytosolic compartment (Figure 2b, last lane) exceeded the intensity of the 10 pmol reference (Figure 2b, second lane), implying a cytosolic concentration of at least 17 µM. In contrast, no signal in the cytosolic fraction could be detected for a non-CPAB formatted control Alphabody. The CPAB cellular uptake was further confirmed by immunofluorescence in HeLa cells that were treated for 30 minutes with CMPX-321A followed by detection of this Alphabody using an anti-V5 antibody. Together, present inventors' data demonstrate that CPAB-formatting of CMPX-152A resulted in an MCL-1-binding Alphabody that efficiently crosses the mammalian cell membrane, without affecting the stability of the Alphabody scaffold nor its ability to bind to MCL-1 (Table 2-3).

**Table 3. Determined affinities of Alphabodies for their targets**

| | CMPX-152A | CMPX-321A | CMPX-383B | CMPX_383B_Gly40 |
|---|---|---|---|---|
| Affinity for MCL-1 | 0.008 nM | 0.015 nM | 0.028 nM | 0.043 nM |
| Affinity for HSA | n.a. | n.a. | 310 nM | 7.5 nM |
| Affinity for MSA | n.a. | n.a. | 2250 nM | 297.5 nM |
| Reported affinities were determined using in-house developed solution ELISA setups, as reported in Material and Methods (Example 1). | | | | |

To evaluate the therapeutic potential of cell-penetrating Alphabodies, present inventors initially screened a panel of MCL-1 dependent cell lines with the CPAB-formatted CMPX-321A Alphabody targeting MCL-1. Present inventors found that several multiple myeloma (MM) cell lines displayed a high sensitivity to CMPX-321A treatment (IC₅₀ < 2 µM), while other cell lines, including colorectal, non-small cell lung, hepatocellular, ovarian and acute myeloid cancer cell lines, were only moderately sensitive or not sensitive at all to MCL-1 inhibition using this anti-MCL-1 CPAB. Interestingly, the high sensitivity of MM cells to MCL-1 inhibition mediated by CMPX-321A appeared to positively correlate with MCL-1 expression levels. Indeed, a survey of RNA expression data from the Broad Institute Cancer Cell Line Encyclopedia (Entrez ID 4170, Accessed 29 Mar. 2020) (M. Ghandi, et al., Next-generation characterization of the Cancer Cell Line Encyclopedia, Nature (2019), doi:10.1038/s41586-019-1186-3; J. Barretina, et al. The Cancer Cell Line Encyclopedia enables predictive modelling of anticancer drug sensitivity, Nature (2012), doi:10.1038/nature11003.) shows that the expression levels of MCL-1 in MM cells is significantly higher than in other cancer types (Figure 2c). These findings prompted present inventors to investigate the cell killing potential of the MCL-1 neutralizing Alphabody against a panel of 33 MM cell lines (Figure 2d). Thirteen MM cell lines from this panel were highly sensitive to CMPX-321A, with IC₅₀ values in the sub-micromolar range and with NCI-H929 being the most sensitive (IC₅₀ of 0.34 µM). Also, 10 MM cell lines showed moderate sensitivity (IC₅₀ values of 1 µM - 2 µM), whilst 6 MM cell lines showed a weaker (IC₅₀ values of 2 µM - 3.31 µM) or no response (IC₅₀ value of 5.03 µM) to CMPX-321A treatment. Four cell lines were excluded from the experiment because they showed a high cell killing percentage (>40%) when treated with the non-MCL-1 binding CPAB-formatted control Alphabody CMPX-322M. Multiple myeloma can be divided in different genetic subsets based on their molecular profile and interestingly, the killing potential of CMPX-321A did not appear to be restricted to a specific genetic subset, indicating that all MM subgroups could benefit from MCL-1 inhibition (Figure 2e). Finally, gene expression analysis revealed that the CMPX-321A potency in MM cell lines is positively correlated with MCL-1 gene expression (Figure 2f). Together, present inventors' data strongly suggest that cell death induced by CMPX-321A in a concentration-dependent manner is a direct consequence of MCL-1-driven inhibition.

### Example 4. Treatment with CPAB CMPX-321A induces BAK activation followed by apoptosis

Next, present inventors sought to confirm that the observed cell killing effect in MM cell lines was mediated by cellular apoptosis. Present inventors treated NCI-H929 cells for 2 and 6 hours with either CMPX-321A or the non-MCL-1 binding CPAB-formatted control Alphabody CMPX-322M and probed for binding of MCL-1 to pro-apoptotic proteins BAK and BIM by co-immunoprecipitation (Figure 3a). Western blot analysis showed that in untreated or CMPX-322M-treated NCI-H929 cells, MCL-1 is associated with BAK and BIM at both time points. In contrast, when these cells were treated with CMPX-321A, a disruption of MCL-1:BAK and MCL-1:BIM complexes was observed after 2h and 6h respectively. Using flow cytometry, present inventors could confirm that cell death in NCI-H929 cells induced by CMPX-321A occurs via a dose-dependent activation of BAK, which is generally considered a hallmark event in apoptosis. (Figure 3b-c). In conclusion, present inventors show that incubation of MM cells with CMPX-321A alleviates MCL-1-mediated sequestering of the pro-apoptotic BAK and, on a longer timescale, BIM. To present inventors' knowledge, Alphabody CMPX-321A is the first non-immunoglobulin protein scaffold capable of translocating across a mammalian cell membrane to neutralize an intracellular target, and to elicit a therapeutically relevant outcome.

### Example 5. Albumin binding increases serum half-life of CMPX-321A without compromising cell-penetration

Due to their typical molecular weight of about 18 kDa, in vivo administered CPAB Alphabodies would be expected to undergo fast renal clearing, suggesting a need to increase their serum half-life to a therapeutically suitable window. Being aware that an increase in molecular weight could impede efficient cell penetration, present inventors leveraged the crystal structure of F. magna Protein G to engineer a minimal albumin binding site (S. Lejon, I. M. Frick, L. Björck, M. Wikström, S. Svensson, Crystal structure and biological implications of a bacterial albumin binding module in complex with human serum albumin, J. Biol. Chem. 279, 42924-42928 (2004)) (Figure 4a). *F. magna* Protein G features a three-helix bundle fold, in which only the two shorter C-terminal α-helices are involved in albumin-binding. Substitution of the larger α-helix of Protein G by the C-helix of CMPX-321A allowed engineering of an albumin binding site with only a modest increase in molecular weight by 3.6 kDa. Finally, present inventors engineered a disulfide bond to tether the two albumin-binding helices to the Alphabody's C-helix to enhance stability. The resulting Alphabody CMPX-383B combines the functionality of CMPX-321A with albumin-mediated half-life extension (HLE) technology, while retaining thermal stability and target affinity (Tables 2-3). Present inventors reasoned that the affinity of the Alphabody for albumin should be carefully tuned to allow for albumin release before translocation into the cell. Indeed, initial immunofluorescence experiments in the presence of mouse serum albumin (MSA) revealed that a too high affinity for MSA impeded timely crossing of the lipid bilayer (Figure 4b, right column). However, by substituting Gly40 in CMPX-383B for Ala present inventors were able to achieve a 40-fold reduction in the affinity of the Alphabody for serum albumin (K_{D} = 7.5 nM vs K_{D} = 310 nM, Table x). Whereas cellular uptake of CMPX-383B in the presence of MSA was slower compared to CMPX-321A (Figure 4b, left column), this effect gradually diminished over time until the 16h time point, where no differences in cellular uptake could be observed (Figure 4b, 3^{rd} row). Thus, it appears that albumin binding slows down, but does not prevent, the cellular uptake of the Alphabodies. The effectiveness of HLE technology to extend the serum half-life of Alphabodies was confirmed by performing a pharmacokinetic (PK) study of administered Alphabody serum concentrations in healthy mice. CD-1 and CB-17 SCID mice were treated with a single intravenously administered bolus injection at 20 mg/kg of repectively CMPX-321A and the half-life extended CMPX-383B. For both studies, Alphabody concentrations in serum and tissue samples were quantified using an in-house developed solution ELISA assay which quantifies the concentration of intact and functionally active Alphabody in the samples. Measurement of serum levels demonstrated that the addition of HLE technology to the Alphabody significantly delays serum clearance (Figure 4c). Indeed, the calculated half-life (t_{1/2}) of CMPX-383B was 76 minutes, which was a 10-fold higher than the t_{1/2} of CMPX-321A (t_{1/2} of 7.6 minutes).

### Example 6. CMPX-383B reduces tumor burden in MM and T- cell leukemia xenograft models in vivo

To further probe the efficacy of Alphabodies with HLE optimization in vivo, present inventors tested the effect of CMPX-383B in xenograft mice tumor models using MM cells, as well as primary T-ALL cells from patients. In a first series of xenograft experiments, two highly sensitive human MM cell lines NCI-H929 and MOLP-8 (in vitro IC₅₀ for CMPX-383B of 0.45 µM and 0.82 µM, respectively) were subcutaneously injected in NSGTM mice (Figure 5a-c). Once the tumors reached an average volume of 80 mm³ (range between 50-100 mm³), treatment with CMPX-383B or vehicle control was initiated. CMPX-383B was administered once daily by intravenous injection at 20 mg/kg for 14 days (Figure 5a). Daily administration of CMPX-383B (20 mg/kg) appeared to be well tolerated by the mice and no toxic side effects by Alphabody CMPX-383B could be observed. During treatment, a significant delay in tumor growth was observed in the CPMX-383B-treated group compared to vehicle-treated mice in both NCI-H929 and MOLP-8 xenograft models (p = 0.002 and 0.007, respectively) (Figure 5b-c). Interestingly, further monitoring after stopping the treatment in MOLP-8 xenografts, revealed a near-significant prolonged delay in tumor growth during the week after the last treatment (p = 0.051) (Figure 8). Thus, present inventors' data show that CMPX-383B has a significant anti-tumor effect in two MM xenograft models.

The promising in vivo effect of a CPAB endowed with HLE capabilities prompted present inventors to study the cell-killing effect of CMPX-383B in multiple myeloma on other hematological malignancies, such as T-ALL. A micro-array gene expression profiling was performed on normal human thymocytes and on a panel of 64 primary T-ALLs (data have been deposited in the GEO with accession number GSE62156) and showed that MCL-1 was significantly higher expressed in most T-ALL subgroups, compared to normal T-cells (Mann-Whitney test; Immature: p = 0.0224 ; TLX: p = 0.0320 ; HOXA: p = 0.0205) (Figure 5d). These findings instigated present inventors to analyze MCL-1 mRNA expression levels in a panel of primary T-ALL patients from the Ghent University Hospital, and to establish a patient-derived xenograft (PDX) from the patient with the highest MCL-1 levels (Figure 5e). To explore the possible advantages of prolonged MCL-1 inhibition by HLE CPBAs in T-ALL patients, present inventors intravenously injected primary T-ALL cells into immunocompromised NSG mice and subsequently started treatment with vehicle or CMPX-383B, according to the treatment schedule depicted in Figure 5f. During the first week of treatment, CMPX-383B (25 mg/kg) was administered daily through tail vein injections. The mice tolerated this dose very well and showed no toxic effects. In order to uncover the full anti-tumorigenic potential of anti-MCLl Alphabody administration, present inventors intensified the treatment schedule by administering CMPX-383B (25 mg/kg) intravenously in the morning followed by a second dose (25 mg/kg) via intraperitoneal injection in the evening. After 14 days of treatment with CMPX-383B, present inventors observed a significant reduction of CD45-positive cells in the blood when compared to the vehicle-treated mice (p = 0.006) (Figure 5f). Collectively, present inventors' data from MM xenograft models and PDX models of T-ALL showed that Alphabody CMPX-383B can significantly reduce MCL-1-mediated tumor burden in vivo.

### Example 7. Structural basis of MCL-1 inhibition by CMPX-383B and of Alphabody half-life extension in serum

Present inventors pursued structural studies of CMPX-383B in complex with either MCL-1 or human serum albumin (HSA) by X-ray crystallography to elucidate the structural basis of the various design principles that were applied towards developing a cell-penetrating Alphabody with specificity for intracellular MCL-1 and favorable serum half-life. For present inventors' structural studies employing MCL-1, present inventors expressed and purified recombinant _{ΔNΔC}MCL-1 (residues 172-320), a minimal construct that contains the BH3 binding groove and which results in higher protein expression and stability (M. L. Stewart, E. Fire, A. E. Keating, L. D. Walensky, The MCL-1 BH3 helix is an exclusive MCL-1 inhibitor and apoptosis sensitizer., Nat. Chem. Biol. 6, 595-601 (2010)). A critical step in the biochemical isolation of the _{ΔNΔC}MCL:CMPX-383B complex concerned the mixing of bacterial cell lysates after expression of the two proteins independently. The structural undertakings involving human serum albumin employed a second variant of CMPX-383B featuring an alternative implementation of the polar core layer. Purified and monodisperse protein samples for _{ΔNΔC}MCL:CMPX-383B and HSA:CMPX-383B complexes led to optimized crystals that diffracted synchrotron X-rays to 2.26 Å and 3.3 Å, respectively (Figures 6 and 7, Table 1).

The crystal structure of the _{ΔNΔC}MCL:CMPX-383B complex revealed the structural basis of MCL-1 inhibition and how CMPX-383B structurally accommodates for the negative design elements featuring a polar core layer (Figure 6). To engage MCL-1, CMPX-383B deploys only the B-helix to bind at the BH3-binding groove of MCL-1 (Figure 6), which is presented by helices α2, α3 and α4 of MCL-1. The six MCL-1 residues that were grafted onto the B-helix of CMPX-383B form the epicenter of the interaction, of which 4 make direct contacts with the hydrophobic platform of MCL-1 (Figure 6a), while Asp_{B3d} and Arg_{B2g} of the Alphabody electrostatically interact with Arg₂₆₃ and Asn₂₆₀, respectively, and the backbone of His₁₅₂ and Val₁₅₃ of _{ΔNΔC}MCL-1 (Figure 6b). The binding of the CMPX-383B B-helix is further secured by interactions at both termini mediated by Glu₄₉, Lys₅₂ and Tyr₇₀, which seal the MCL-1 groove otherwise used to sequester anti-apoptotic mediators. Importantly, the B-helix of CMPX-383B superimposes very well with the structure of the inhibitory MCL-1 helix SAHB_{D} in complex with MCL-1 (R.M.S.D. of 0.713 Å for 17 aligned Cα-atoms) (Figure 6g), including the hallmark arching imposed onto the inhibiting helix by MCL-1 (Figure 6 e-f). Indeed, the MCL-1-binding B-helix of CMPX-383B displays a curvature of almost 30° towards its C-terminus that coincides with a slight widening of the Alphabody core initiated at the polar core layer. The engineered polar core layer serves a twofold purpose to facilitate interaction of the core Asp_{B3d} of CMPX-383B with Arg₂₆₃ of _{ΔNΔC}MCL-1, and to mediate compliance with the conformational criteria imposed by the binding target. Collectively, such direct structural evidence illustrates how the Alphabody scaffold can be tailored to the particular structural landscape of the molecular target to bind with high specificity. The crystal structure of the HSA:CMPX-383B complex allowed assessment of the possible structure-function consequences of HLE technology for the Alphabody scaffold (Figure 6). Similar to the grafted MCL-1 inhibitory signature on the B-helix, present inventors observed that the appended D- and E-helices of CMPX-383B are virtually superimposable with the F. magna protein G template (R.M.S.D. of 0.49 Å over 48 Cα atoms) (Figure 6h), providing direct evidence that the HLE technology allows an Alphabody to bind domain II of the albumin molecule using an extensive network of electrostatic interactions (Figure 6c-d). These interactions are reinforced by the burial of Phe₁₁₈ and, to a lesser extent, Leu₁₂₂ of the Alphabody into a hydrophobic pocket of HSA. Taken together, these two crystallographic structures highlight the engineerability and modularity of the Alphabody scaffold to accommodate tailored binding requirements to enhance pharmacokinetic performance.

## Claims

1. A polypeptide capable of specifically binding myeloid cell leukemia 1 (MCL-1) comprising at least one Alphabody structure having the general formula
HRS1-L1-HRS2-L2-HRS3,
- wherein each of HRS1, HRS2 and HRS3 is independently a heptad repeat sequence (HRS) comprising 2 to 7 consecutive but not necessarily identical heptad repeat units,
- wherein said heptad repeat units are 7-residue (poly)peptide fragments represented as 'abcdefg' or 'defgabc', wherein the symbols 'a' to 'g' denote conventional heptad positions,
- wherein at least 50% of all heptad a- and d-positions are occupied by isoleucine residues,
- wherein each HRS starts and ends with an aliphatic or aromatic amino acid residue located at a heptad a-position or d-position,
- wherein each of L1 and L2 are independently a linker fragment, which covalently connect HRS1 to HRS2 and HRS2 to HRS3, respectively,
**characterized in that**
- HRS2 comprises a MCL-1 binding region comprising a sequence LRXVGDXV (SEQ ID NO: 1), wherein X can be any amino acid;
- the polypeptide comprises an albumin binding region conjugated to the C-terminus of HRS3, wherein said albumin binding region comprises a sequence SDFYFXXINKA (SEQ ID NO: 2) and a sequence TXEXVXALKXXILXAH (SEQ ID NO: 3), wherein X can be any amino acid;
- HRS1, HRS2 and HRS3 form a triple-stranded, anti-parallel, alpha-helical coiled coil; and
- HRS 1, HRS2, HRS3 and the albumin binding region together form a five-stranded alpha-helical bundle.

2. The polypeptide according to claim 1, wherein the albumin binding region comprises a sequence SDFYFXXINKAKTXEXVXALKXXILXAH (SEQ ID NO: 4), preferably a sequence SDFYFXXINKAKTCEAVXALKXXILXAH (SEQ ID NO: 5), wherein X can be any amino acid.

3. The polypeptide according to claim 1 or 2, further comprising a peptide for facilitating cellular entry of the polypeptide, wherein said peptide comprises a sequence (RP)ₙ, wherein n is an integer from 6 to 9.

4. The polypeptide according to claim 3, wherein said peptide for facilitating cellular entry of the polypeptide is conjugated to the N- and/or C-terminus of the polypeptide as defined in claim 1 or 2.

5. A nucleic acid sequence encoding the polypeptide as set forth in any one of claims 1 to 4.

6. A pharmaceutical composition comprising the polypeptide according to any one of claims 1 to 4, and a pharmaceutically acceptable carrier.

7. The polypeptide according to any one of claims 1 to 4, or the pharmaceutical composition according to claim 6 for use as a medicament.

8. The polypeptide according to any one of claims 1 to 4, or the pharmaceutical composition according to claim 6 for use in the treatment of an MCL-1 mediated disease or disorder, preferably a neoplastic disease or an autoimmune disease.
